# FASCICULE DE BREVET EUROPEEN

(11) **EP 3 727 390 B1**
(45) Date de publication et mention de la délivrance du brevet: **22.03.2023**
(21) Numéro de dépôt: 18826025.1
(22) Date de dépôt: 20.12.2018
(51) Int. Cl.: C07D 487/04, C07D 487/20, A61K 31/519, A61P 17/00

(54) **COMPOSES INHIBITEURS DE MTOR**
MTOR HEMMER
MTOR INHIBITORS

(30) Priorité: 21.12.2017 FR 1771402
(43) Date de publication de la demande: 28.10.2020
(73) Titulaire: Galderma Research & Development, 06410 Biot (FR)
(72) Inventeur: CLARY, Laurence, 06480 La Colle Sur Loup (FR); FOURNIER, Jean-François, 06600 Antibes (FR); OUVRY, Gilles, Abingdon, OXI143TA Oxfordshire (FR); BHURRUTH-ALCOR, Yushma, Ashburn, Virginie 20147 (US); THOREAU, Etienne, 06460 Saint Vallier De Thiey (FR); TOMAS, Loïc, 1814 La-Tour-de-Peilz (CH)
(74) Mandataire: Nederlandsch Octrooibureau
(86) Numéro de dépôt international: PCT/EP2018/086074
(87) Numéro de publication internationale: WO 2019/122065

(56) Documents cités:
- EP-B1- 2 705 181
- WO-A1-2015/074135
- WO-A2-2007/061737
- WO-A2-2012/154695

## Description

La présente invention concerne de nouveaux composés inhibiteurs de la sérine/thréonine kinase mTOR (« mechanistic target of rapamycin » ou cible fonctionnelle de la rapamycine, également connue sous le nom de FRAP, RAFT, RAPT, SEP). Elle concerne également des compositions les comprenant, leurs procédés de préparation et leurs utilisations dans des compositions en tant que médicament.

La protéine kinase mTOR est le centre catalytique de deux complexes multiprotéiques fonctionnellement distincts, conservés chez tous les eucaryotes et nommés mTORC1 et mTORC2 (Dunlop et al., « Mammalian target of rapamycin complex 1: signalling inputs, substrates and feedback mechanisms », 2009 ; Guertin et al., « The pharmacology of mTOR inhibition », 2009). Lorsqu'elle est associée à Raptor (regulatory associated protein of TOR) et à mLST8 (mammalian lethal with sec 13 protein 8), mTOR forme le complexe mTORC1. Ce complexe interagit avec Deptor (DEP domaincontaining mTOR-interacting protein), FKBP38 et PRAS40 (prolinerich Akt substrate of 40 kDa), régulateurs négatifs de mTORC1. Pour former mTORC2, mTOR interagit avec les protéines Rictor (rapamycininsensitive companion of TOR), Sin1 (stress-activated map kinaseinteracting protein 1) et mLST8. De plus, mTORC2 s'associe aussi avec Deptor, qui réprime son activité, ainsi qu'avec PPR5/Protor, dont la fonction demeure inconnue. Lorsqu'elle est liée à FKBP12, la rapamycine inhibe spécifiquement mTORC1.
mTOR est notamment connue pour réguler la prolifération cellulaire, la croissance cellulaire, la mobilité cellulaire, la survie cellulaire, la biosynthèse des protéines et la transcription.

Il a été montré que les perturbations de la voie de signalisation de mTOR sont à l'origine de plusieurs maladies, en particulier différents types de cancer et des hamartomes multiples.

On connaît par exemple le document brevet WO2007061737 qui divulgue des composés bicycliques inhibiteurs de mTOR. Ils sont utilisés dans le traitement du cancer tel que le cancer du sein, cancer du poumon, cancer du poumon non à petites cellules, cancer du rein, carcinome rénal, cancer de la prostate, cancer du sang, cancer du foie, cancer de l'ovaire, cancer de la thyroïde, cancer de l'endomètre, lymphome, carcinome des cellules rénales, ou encore lymphome du manteau.

On connaît également le document brevet WO2009117482 qui décrit plus particulièrement des sels et autres polymorphes de composés bicycliques inhibiteurs de mTOR, également utilisés dans le traitement du cancer, du même type que ceux décrits dans WO2007061737.

EP2705181B1 et WO2012154695 décrivent des composés de pyrimidine condensé comme inhibiteurs de mTOR.

Les composés inhibiteurs de mTOR décrits dans WO2015074135 avant une structure pyrrole-pyrimidine et peuvent être utilisés dans le traitement du psoriasis.

La rapamycine, inhibiteur de mTOR, est connue depuis longtemps pour ses propriétés immunosuppressives. Elle a néanmoins montré un succès thérapeutique limité lorsqu'elle est administrée par voie systémique à des patients atteints de psoriasis. Aussi, des données récentes ont montré que la voie de signalisation mTOR est hyperactivée dans la peau psoriasique lésionnelle, ce qui pourrait contribuer à la maladie en interférant avec la maturation des kératinocytes. L'effet du traitement topique de la rapamycine dans un modèle de souris psoriasique induite par l'imiquimod a été étudié (Bürger et al., «Blocking mTOR Signalling with Rapamycin Améliorâtes Imiquimod-induced Psoriasis in Mice», 2017). L'analyse immunohistologique a révélé que la rapamycine non seulement empêchait l'activation de la voie de signalisation mTOR (niveaux de P-mTOR et de P-S6), mais presque normalisait l'expression des marqueurs de différenciation épidermique. En outre, l'afflux de cellules immunitaires innées dans les ganglions lymphatiques drainants a été partiellement réduit par le traitement à la rapamycine. Ces données soulignent le rôle de la voie de signalisation mTOR dans la pathogenèse du psoriasis, et soutiennent l'étude de l'inhibition topique de la mTOR en tant que nouvelle stratégie anti-psoriasique.

Il existe donc un réel besoin de développer des traitements en particulier topiques de patients atteints de maladies telles que le psoriasis.

Considérant ce qui précède, un problème que se propose de résoudre l'invention est de proposer de nouveaux inhibiteurs de mTOR pour améliorer notamment le traitement de maladies prolifératives ou inflammatoires à médiation immunitaire de la peau.

La Demanderesse a développé de nouveaux composés inhibiteurs de mTOR.

Ainsi, la présente invention a pour objet un composé inhibiteur de mTOR de formule générale (I) 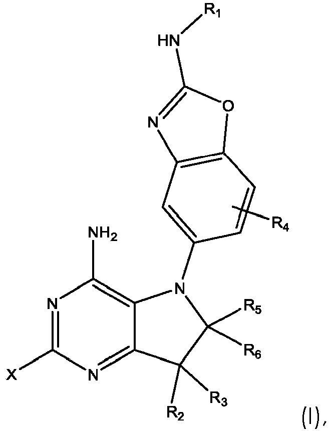 ou un de ses sels pharmaceutiquement acceptables, dans laquelle chaque variable est telle que définie et décrite ci-après.

L'invention a également pour objet une composition comprenant, dans un milieu physiologiquement acceptable, un composé inhibiteur de mTOR de formule (I) selon l'invention ou un de ses sels pharmaceutiquement acceptables. Elle est destinée à être utilisée en tant que médicament, en particulier dans le traitement des maladies impliquant une enzyme mTOR à activité sérine-thréonine kinase et notamment dans le traitement des affections dermatologiques liées à un trouble de la kératinisation avec une composante proliférative, inflammatoire et/ou immuno-allergique telles que par exemple le psoriasis, la dermatite atopique, la kératose actinique, ou l'acné, préférentiellement la dermatite atopique, plus préférentiellement la composante inflammatoire de la dermatite atopique et encore plus préférentiellement le traitement par voie topique de la composante inflammatoire de la dermatite atopique.

L'invention sera mieux comprise à la lecture de la description qui suit, rédigée au regard des dessins annexés, dans lesquels :

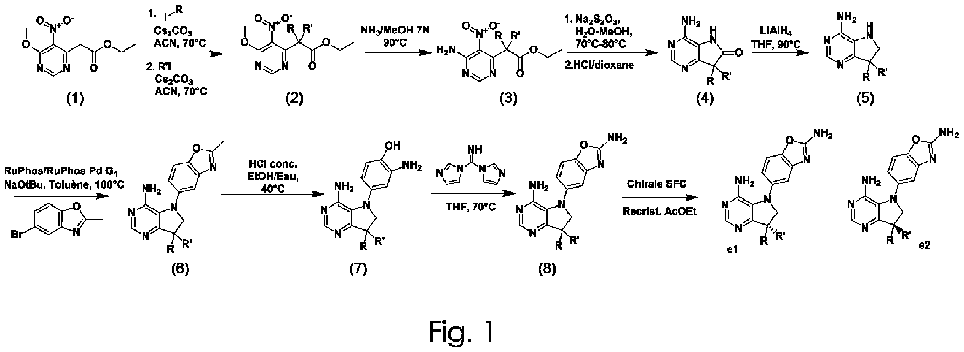

- la Figure 1 représente une voie de synthèse des composés 5-(4-Amino-7-isobutyl-7-methyl-6,7-dihydro-5H-pyrrolo[3,2-d] pyrimidin-5-yl)benzo[d]oxazol-2-amine (énantiomère 1 - e1) et 5-(4-Amino-7-isobutyl-7-methyl-6,7-dihydro-5H-pyrrolo [3,2-d] pyrimidin-5-yl) benzo [d] oxazol-2-amine (énantiomère 2 - e2) ; et

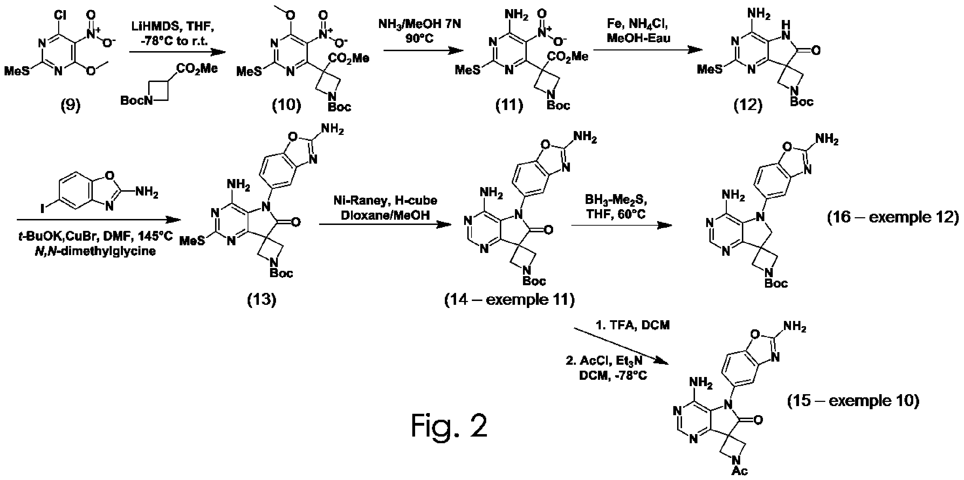

- la Figure 2 représente une voie de synthèse des composés 1-Acetyl-4'-amino-5'-(2-aminobenzo[d]oxazol-5-yl)spiro[azetidine-3,7'-cyclopenta[d]pyrimidin]-6'(5'H)-one (exemple 10), tert-butyl-4'-amino-5'-(2-aminobenzo[d]oxazol-5-yl)-6'-oxo-5',6'-dihydrospiro[azetidine-3,7'-pyrrolo[3,2-d]pyrimidine]-1-carboxylate (exemple 11), et tert-butyl-4'-amino-5'-(2-aminobenzo[d]oxazol-5-yl)-5',6'-dihydrospiro[azetidine-3,7'-pyrrolo[3,2-d]pyrimidine]-1-carboxylate (exemple 12).

La présente invention concerne des nouveaux composés inhibiteurs de mTOR ou un de leurs sels pharmaceutiquement acceptables.

Par inhibiteur de mTOR, on entend des composés qui vont réguler négativement c'est-à-dire réduire, bloquer voire supprimer l'activation de la voie de signalisation mTOR, en faisant compétition, avantageusement de manière sélective, avec les substrats au niveau de mTORC1 et/ou mTORC2 ou en modifiant le site actif de ces enzymes qui ne peuvent ainsi plus catalyser un substrat donné.

Les composés selon l'invention peuvent être représentés par la formule générale (I) 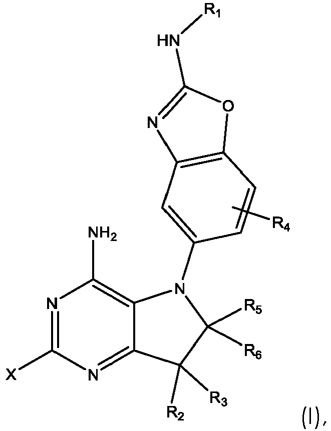 ou un de leurs sels pharmaceutiquement acceptables, dans laquelle :
R₁ représente un atome d'hydrogène, un radical alkyle en C₁-C₃, cyclopropyle ou acyle ;
R₂ et R₃ représentent, indépendamment l'un de l'autre, un atome d'hydrogène, un atome d'halogène choisi parmi Cl ou F, un radical alkyle en C₁-C₆ linéaire ou ramifié, interrompu ou non par un hétéroatome O, S, ou -NR₇, et non substitué ou substitué par un cycloalkyle ou un hétérocycloalkyle en C₃-C₅ ou un cycle aromatique/hétérocycle non substitué ou mono- ou poly-substitué par un atome d'halogène choisi parmi CI, F ou un radical -OH, méthyle (Me), -OMe, ou forment ensemble un cycle ou un hétérocycloalkyle en C₃-C₆ ;
étant entendu que R₂ et R₃ ne représentent pas tous deux un atome d'halogène ;
   avec R₇ représentant un atome d'hydrogène, un radical alkyle en C₁-C₃, acyle, ou carboxyalkyle en C₁-C₄ ;
R₄ représente un atome d'hydrogène, un atome d'halogène choisi parmi F, Cl, Br, un radical -OR₈, alkyle en C₁-C₃, cyclopropyle ;
   avec R₈ représentant un atome d'hydrogène, un radical alkyle en C₁-C₃, cyclopropyle ou acyle ;
R₅ et R₆ représentent, indépendamment l'un de l'autre, un atome d'hydrogène, un radical alkyle en C₁-C₃, cyclopropyle ou forment ensemble un carbonyle ou un cycle en C₃-C₄ ; et
X représente un atome d'hydrogène, ou un radical -NH₂, -OH, méthyle.

Selon la présente invention, par alkyle, on entend un radical linéaire ou ramifié ayant par exemple de 1 à 6 (en C₁-C₆). ou de 1 à 3 (en C₁-C₃), atomes de carbone, avantageusement les radicaux méthyle, éthyle, propyle, isopropyle, butyle, tertiobutyle, 2-méthylbutyle, pentyle, 2-méthylpentyle, hexyle.

Par acyle, on entend un radical obtenu en enlevant le groupement hydroxyle d'un acide carboxylique ; le groupement acyle correspondant à un acide carboxylique de formule -RCOOH aura pour formule -RCO, où l'atome de carbone et celui d'oxygène sont liés par une double liaison (groupement carbonyle).

Par cycloalkyle, on entend un radical cycloalkyles ayant de 3 à 6 atomes de carbone avantageusement choisi parmi cyclopropyle, cyclopentyle, cyclohexyle.

Par hétérocycloalkyle ou hétérocycle, on entend par exemple un radical pipéridino, morpholino, pyrrolidino ou pipérazino.

Par cycle aromatique, on entend un radical cyclique plan et possédant (4n + 2) électrons délocalisés, n étant le nombre de cycles constituant le radical ; si le cycle contient des éléments autres que le carbone et l'hydrogène, on parle d'hétérocycle aromatique.

Lorsque les composés selon l'invention se présentent sous forme d'un sel pharmaceutiquement acceptable, il s'agit de préférence d'un sel obtenu à partir d'une base ou d'un acide non toxiques.

Le terme « sel pharmaceutiquement acceptable » se réfère aux sels qui sont, dans le cadre d'un bon jugement médical, appropriés pour une utilisation en contact avec les tissus humains et animaux inférieurs sans toxicité excessive, irritation, réponse allergiques et similaires et sont proportionnels à un rapport bénéfice/risque raisonnable. Les sels pharmaceutiquement acceptables sont bien connus dans l'état de l'art. Les sels pharmaceutiquement acceptables des composés de la présente invention comprennent ceux dérivés d'acides et de bases inorganiques et organiques appropriés.

Lorsque le composé de la présente invention est acide, son sel correspondant peut être préparé à partir de bases non toxiques pharmaceutiquement acceptables, comprenant des bases inorganiques et des bases organiques.

Les sels dérivés de ces bases inorganiques comprennent l'aluminium, l'ammonium, le calcium, le cuivre, le fer, le lithium, le magnésium, le manganèse, le potassium, le sodium et les sels similaires. Les sels d'ammonium, de calcium, de magnésium, de potassium et de sodium sont particulièrement préférés.

Les sels dérivés de bases organiques non toxiques pharmaceutiquement acceptables comprennent des sels d'amines primaires, secondaires et tertiaires, ainsi que des amines cycliques et des amines substituées telles que des amines substituées naturellement et synthétisées.

D'autres bases organiques non toxiques pharmaceutiquement acceptables à partir desquelles des sels peuvent être formés comprennent des résines échangeuses d'ions telles que, par exemple, l'arginine, bétaïne, caféine, choline, N',N'-dibenzyléthylènediamine, diéthylamine, 2-diéthylaminoéthanol, 2-diméthylaminoéthanol, éthanolamine, N,N-diéthyléthanolamine, éthylènediamine, N-éthylmorpholine, N-éthylpipéridine, glucamine, glucosamine, histidine, hydrabamine, isopropylamine, lysine, méthylglucamine, morpholine, pipérazine, pipéridine, résines polyamine, procaïne, purines, théobromine, triéthylamine, triméthylamine, tripropylamine, trométhamine et similaires.

Lorsque le composé de la présente invention est basique, son sel correspondant peut être préparé à partir d'acides non toxiques pharmaceutiquement acceptables, comprenant des acides inorganiques et organiques.

De tels acides comprennent, par exemple, un acide acétique, benzènesulfonique, benzoïque, camphorsulfonique, citrique, éthanesulfonique, fumarique, gluconique, glutamique, bromhydrique, chlorhydrique, iséthionique, lactique, maléique, malique, mandélique, méthanesulfonique, mucique, nitrique, pamoïque, pantothénique, phosphorique, succinique, sulfurique, tartrique, p-toluènesulfonique et similaires. Les acides citrique, bromhydrique, chlorhydrique, maléique, phosphorique, sulfurique et tartrique sont particulièrement préférés.

Selon un mode de réalisation de l'invention, le sel pharmaceutiquement acceptable est choisi parmi la trométhamine, le sodium, le calcium et la L-arginine.

Selon un autre mode de réalisation de l'invention, le sel est choisi parmi le magnésium, potassium, N,N-diéthyléthanolamine, N-méthyle-D-glucamine ou pipérazine.

Dans certains modes de réalisation, le sel est sous forme de sel d'hydrate ou de solvate.

Dans certains modes de réalisation, le sel est sensiblement sous forme amorphe.

Dans certains modes de réalisation, le sel est essentiellement sous forme cristalline.

Dans certains modes de réalisation, le sel est au moins à environ 95% en poids cristallin.

Dans certains modes de réalisation, le sel est sensiblement sous forme cristalline unique.

Selon la présente invention, les composés de formule (I), ou un de leurs sels pharmaceutiquement acceptables, préférés sont ceux dans laquelle :
R₁ représente un atome d'hydrogène ;
R₂ et R₃ représentent, indépendamment l'un de l'autre, un atome d'hydrogène, un radical alkyle en C₁-C₄ linéaire ou ramifié, interrompu ou non par un hétéroatome S ou -NR₇, ou forment ensemble un hétérocycloalkyle en C4 ;
   avec R₇ représentant un radical acyle, carboxytertiobutyle ;
R₄ représente un atome d'hydrogène ;
R₅ et R₆ représentent, indépendamment l'un de l'autre, un atome d'hydrogène, un groupe méthyle, ou forment ensemble un carbonyle ; et
X représente un atome d'hydrogène.

Plus préférentiellement, les composés de formule (I), ou un de leurs sels pharmaceutiquement acceptables, sont ceux dans laquelle :
R₁ représente un atome d'hydrogène ;
R₂ représente un groupe méthyle ;
R₃ représente un radical alkyle en C₁-C₄ linéaire ou ramifié, tel que par exemple un méthyle, éthyle, propyle, isopropyle, butyle, isobutyle ou tertiobutyle, interrompu ou non par un hétéroatome S ;
R₄ représente un atome d'hydrogène ;
R₅ et R₆ représentent un atome d'hydrogène ; et
X représente un atome d'hydrogène.

Encore plus préférentiellement, les composés de formule (I), ou un de leurs sels pharmaceutiquement acceptables, sont ceux dans laquelle :
R₁ représente un atome d'hydrogène ;
R₂ représente un groupe méthyle ;
R₃ représente un groupe isobutyle, les deux énantiomères R et S étant représentés,
R₄ représente un atome d'hydrogène ;
R₅ et R₆ représentent un atome d'hydrogène ; et
X représente un atome d'hydrogène.

Parmi les composés de formule (I) entrant dans le cadre de la présente invention, on peut notamment citer les composés suivants :
- 5-(4-Amino-7-isobutyl-7-methyl-6,7-dihydro-5H-pyrrolo[3,2-d]pyrimidin-5-yl)benzo[d]oxazol-2-amine (énantiomère 1) ;
- 5-(4-Amino-7-isobutyl-7-methyl-6,7-dihydro-5H-pyrrolo[3,2-d]pyrimidin-5-yl)benzo[d]oxazol-2-amine (énantiomère 2) ;
- 5-(2-Amino-benzooxazol-5-yl)-7-isobutyl-6,7-dihydro-5H-pyrrolo[3,2-d]pyrimidin-4-ylamine ;
- 5-(2-Amino-benzooxazol-5-yl)-7-methyl-7-methylsulfanylmethyl-6,7-dihydro-5H-pyrrolo[3,2-d]pyrimidin-4-ylamine ;
- 5-(4-Amino-7,7-diethyl-6,7-dihydro-5H-pyrrolo[3,2-d]pyrimidin-5-yl)benzo[d]oxazol-2-amine ;
- 5-(2-Amino-benzooxazol-5-yl)-7,7-dimethyl-6,7-dihydro-5H-pyrrolo[3,2-d]pyrimidin-4-ylamine ;
- 5-(2-Amino-benzooxazol-5-yl)-7-methyl-7-propyl-6,7-dihydro-5H-pyrrolo[3,2-d]pyrimidin-4-ylamine ;
- 5-(2-Amino-benzooxazol-5-yl)-7,7-diethyl-6-methyl-6,7-dihydro-5H-pyrrolo[3,2-d]pyrimidin-4-ylamine ;
- 4-Amino-5-(2-amino-benzooxazol-5-yl)-7,7-dimethyl-5,7-dihydro-pyrrolo[3,2-d]pyrimidin-6-one ;
- 1-Acetyl-4'-amino-5'-(2-aminobenzo[d]oxazol-5-yl)spiro[azetidine-3,7'-cyclopenta[d]pyrimidin]-6'(5'H)-one;
- Tert-butyl-4'-amino-5'-(2-aminobenzo[d]oxazol-5-yl)-6'-oxo-5',6'-dihydrospiro[azetidine-3,7'-pyrrolo [3,2-d]pyrimidine]-1-carboxylate;
- Tert-butyl-4'-amino-5'-(2-aminobenzo[d]oxazol-5-yl)-5',6'-dihydrospiro[azetidine-3,7'-pyrrolo[3,2-d]pyrimidine]-1-carboxylate.

La présente invention a également pour objet une composition comprenant, dans un milieu physiologiquement acceptable, un composé de formule (I) selon l'invention tel que défini ci-avant ou un de ses sels pharmaceutiquement acceptables.

Un milieu pharmaceutiquement acceptable désigne un milieu compatible et adapté pour une utilisation en contact avec des cellules humaines et animales, en particulier avec la peau, les muqueuses et/ou les phanères, sans toxicité, irritation, réponse allergique indue et similaires, et proportionné à un rapport avantage/risque raisonnable.

Un milieu pharmaceutiquement acceptable selon l'invention peut comprendre tout adjuvant connu et utilisé dans le domaine pharmaceutique, compatible avec les composés inhibiteurs de mTOR selon l'invention.

A titre d'exemple on peut citer des solvants, tampons, aromatisants, liants, agents chélatants, agents tensioactifs, épaississants, lubrifiants, gélifiants, humectants, hydratants, conservateurs, antioxydants, agents apaisants, agents pro-pénétrants, colorants, parfums et similaires, ou leur mélange.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée. Leur concentration est également choisie de sorte à ce qu'elle ne nuise pas aux propriétés avantageuses des composés selon l'invention.

Le composé selon la présente invention, et la composition le comprenant, peuvent être administrés par voie orale, rectale, topique, ou parentérale (sous-cutanée, intramusculaire ou intraveineuse). Ils sont de préférence administrés par voie orale ou topique, plus préférentiellement topique.

Les compositions selon l'invention peuvent se présenter sous forme liquide, solide ou encore de gaz.

Par voie orale, la composition, peut se présenter sous forme de comprimés, de gélules, de dragées, de sirops, de suspensions, de solutions, de poudres, de granulés, d'émulsions, de suspensions de microsphères ou nanosphères ou de vésicules lipidiques ou polymériques permettant une libération contrôlée.

Par voie parentérale, la composition peut se présenter sous forme de solutions ou suspensions pour perfusion ou pour injection.

De préférence, la composition pharmaceutique est conditionnée sous une forme convenant à une application par voie topique.

Par voie topique, les compositions, qui sont donc plus particulièrement destinées au traitement de la peau et des muqueuses, peuvent se présenter sous forme liquide, pâteuse, ou solide, et plus particulièrement sous forme d'onguents, de crèmes, de laits, de pommades, de poudres, de tampons imbibés, de syndets, de solutions, de gels, de sprays, de mousses, de lotions, de suspensions, de sticks, de shampoings, ou de bases lavantes. Elle peut également se présenter sous forme de suspensions de microsphères ou nanosphères, de vésicules lipidiques ou polymériques, de patches polymériques ou gélifiés, ou d'hydrogels permettant une libération contrôlée des composés actifs. Ces compositions par voie topique peuvent par ailleurs se présenter soit sous forme anhydre, soit sous une forme aqueuse.

La composition selon l'invention comprend préférentiellement entre 0,001% et 5% dudit composé de formule (I) ou un de ses sels pharmaceutiquement acceptables, en poids du poids total de la composition.

La quantité réellement administrée à mettre en oeuvre selon l'invention dépend de l'effet thérapeutique recherché, et peut donc varier dans une large mesure. L'homme de l'art, en particulier le médecin peut aisément, sur la base de ses connaissances générales déterminer les quantités appropriées.

La composition selon l'invention peut comprendre au moins un autre ingrédient actif.

L'ingrédient actif additionnel est préférentiellement choisi parmi le groupe comprenant les antibiotiques, antibactériens, antiviraux, antiparasitaires, antifongiques, anesthésiques, analgésiques, antiallergiques, rétinoïdes, anti-radicaux libres, antiprurigineux, antihistaminiques, immunosuppresseurs, corticostéroïdes, agents kératolytiques, immunoglobulines intraveineuses, anti-angiogéniques, anti-inflammatoires et/ou un mélange de ceux-ci.

Plus préférentiellement, l'ingrédient actif additionnel est connu pour son efficacité dans le traitement des affections dermatologiques liées à un trouble de la kératinisation avec une composante proliférative, inflammatoire et/ou immuno-allergique telles que le psoriasis, la dermatite atopique, la kératose actinique, ou l'acné.

A titre d'exemple d'ingrédient actif additionnel, on peut citer des ingrédients choisis parmi la bétaméthasone dipropionate glycol, clobétasol 17-propionate, halobétasol propionate, amcinonide, desoximétasone, diflucortolone valérate, fluocinonide, halcinonide, mométhasone furoate, triamcinolone acétonide, bétamethasone valérate, clobétasone 17-butyrate, désonide, hydrocortisone 17-valérate, prédnicarbate, hydrocortisone, hydrocortisone acétate, calcipotriol, calcitriol, adapalène, péroxyde de benzoyle, clindamycine et érythromycine.

La présente invention concerne de nouveaux composés inhibiteurs de mTOR de formule (I).

Ainsi, la présente invention a pour objet les composés de formule (I) tels que décrits ci-dessus destinés à être utilisés à titre de médicament.

L'invention a également pour objet une composition selon l'invention, pour son utilisation en tant que médicament, en particulier dans le traitement des maladies impliquant une enzyme mTOR à activité sérine-thréonine kinase chez un patient.

Les termes « traiter » ou « traitement », tels qu'utilisés dans la présente invention, se rapportent à l'inversion, à l'atténuation, à l'inhibition de la progression, au retard de l'apparition, à l'amélioration et/ou au soulagement partiel ou total d'une maladie ou d'un trouble, ou d'un ou plusieurs symptômes de la maladie ou du trouble, tels que décrits ci-après. Dans certains modes de réalisation, le traitement peut être administré après qu'un ou plusieurs symptômes se sont développés. Dans certains modes de réalisation particuliers, le traitement peut être administré en tant que mesure préventive, pour prévenir ou arrêter la progression d'une maladie ou d'un trouble. Dans ce contexte, la « prévention » désigne une réduction du risque d'acquisition d'une maladie ou d'un trouble déterminé. Dans d'autres modes de réalisation, le traitement peut être administré en l'absence de symptômes. Par exemple, le traitement peut être administré à un individu prédisposé avant l'apparition des symptômes (par exemple à la lumière d'antécédents de symptômes et/ou de facteurs génétiques ou autres facteurs de prédisposition). Le traitement peut également être poursuivi après la disparition des symptômes, par exemple pour prévenir ou retarder leur réapparition. Ainsi, dans certains modes de réalisation, le terme « traitement » comprend la prévention d'une rechute ou d'une récurrence d'une maladie ou d'un trouble.

Le terme « patient », tel qu'utilisé dans la présente invention, désigne un mammifère et inclut des sujets humains et animaux, de préférence un humain.

La composition selon l'invention est plus particulièrement destinée à être utilisée dans le traitement des affections dermatologiques liées à un trouble de la kératinisation avec une composante proliférative, inflammatoire et/ou immuno-allergique.

Les affections dermatologiques liées à un trouble de la kératinisation avec une composante proliférative, inflammatoire et/ou immuno-allergique comprennent les troubles ou désordres de la kératinisation portant sur la prolifération cellulaire notamment, les acnés vulgaires, comédoniennes, polymorphes, rosacées, les acnés nodulokystiques, conglobata, les acnés séniles, les acnés secondaires telles que l'acné solaire, médicamenteuse ou professionnelle, les autres troubles de la kératinisation, notamment les ichtyoses, les états ichtyosif ormes, la maladie de Darier, les keratodermies palmoplantaires, les leucoplasies et les états leucoplasiformes, le lichen cutané ou muqueux (buccal), les autres affections dermatologiques liées à un trouble de la kératinisation avec une composante inflammatoire et/ou immuno-allergique notamment, toutes les formes de psoriasis qu'il soit cutané, muqueux ou unguéal, et même le rhumatisme psoriatique, ou encore l'atopie cutanée, telle que la dermatite atopique (ou eczéma atopique) ou l'atopie respiratoire ou encore l'hypertrophie gingivale, toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les lésions ou proliférations pouvant être induites par les ultra-violets notamment dans le cas des kératoses actiniques, épithélioma baso et spinocellulaires.

Plus préférentiellement, la composition selon l'invention est destinée à être utilisée dans le traitement des affections dermatologiques liées à un trouble de la kératinisation avec une composante proliférative, inflammatoire et/ou immuno-allergique telles que le psoriasis, la dermatite atopique, la kératose actinique, ou l'acné, encore plus préférentiellement la dermatite atopique.

De manière particulièrement avantageuse, la composition selon l'invention est destinée à être utilisée dans le traitement de la composante inflammatoire de la dermatite atopique, et préférentiellement le traitement par voie topique de la composante inflammatoire de la dermatite atopique.

Par « composante inflammatoire de la dermatite atopique », on entend une inflammation impliquant des lymphocytes CD4+, des éosinophiles, des mastocytes et des cytokines Th2.

La présente invention a également pour objet les procédés de préparation des composés de formule (I), en particulier selon les schémas réactionnels donnés aux Figures 1 et 2.

On va maintenant donner, à titre d'illustration plusieurs exemples d'obtention de composés actifs de formule (I) selon l'invention et des résultats d'activité inhibitrice.

### Exemples 1 et 2 : voie de synthèse des composés 5-(4-amino-7-isobutyl-7-methyl-6,7-dihydro-5H-pyrrolo[3,2-d]pyrimidin-5-yl)benzo[d]oxazol-2-amine (énantiomère 1 - e1) et 5-(4-amino-7-isobutyl-7-methyl-6,7-dihydro-5H-pyrrolo[3,2-d]pyrimidin-5-yl)benzo[d]oxazol-2-amine (énantiomère 2-e2) telle qu'illustrée par la Figure 1

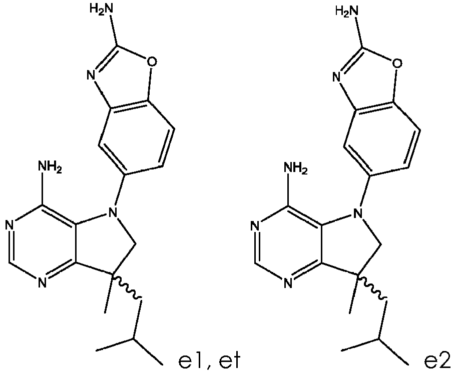

a) Ethyl-2-(6-methoxy-5-nitropyrimidin-4-yl)-2,4-dimethylpentanoate 1-iodo-2-methyl-propane (13.12 ml; 0.11 mol; 1.10 eq.) est ajouté sur une suspension d'ester éthylique d'acide (6-methoxy-5-nitro-pyrimidin-4-yl)-acétique (25.00 g; 0.10 mol; 1.00 eq.) (1) et de carbonate de césium (37.15 g; 0.11 mol; 1.10 eq.) dans l'acétonitrile (250.00 ml). Le milieu réactionnel est chauffé à 70°C pendant 12 heures. Du carbonate de césium (37.15 g; 0.11 mol; 1.10 eq.) suivi de iodométhane (7.74 ml; 0.12 mol; 1.20 eq.) sont ensuite ajoutés et le milieu réactionnel est chauffé à 70°C pendant 5 heures. Après retour à température ambiante, le milieu réactionnel est filtré pour éliminer le carbonate de césium puis le filtrat est concentré sous vide. Le résidu (33.8 g) est chromatographié sur gel de silice avec dépôt solide (colonne puriFlash IR-50SI/800G, puriFlash) élué à l'heptane/acétate d'éthyle (98/2 à 90/10). L'ester éthylique d'acide 2-(6-methoxy-5-nitro-pyrimidin-4-yl)-2,4-dimethyl-pentanoïque (21.3 g; 66%) (2) est obtenu sous la forme d'une huile jaune claire.

### b) Ethyl-2-(6-amino-5-nitropyrimidin-4-yl)-2,4-dimethylpentanoate

Dans un autoclave, de l'ammoniaque 7N dans du méthanol (216.00 ml) est ajouté sur une solution d'ester éthylique d'acide 2-(6-methoxy-5-nitro-pyrimidin-4-yl)-2,4-dimethyl-pentanoïque (21.26 g; 0.07 mol; 1.00 eq.) (2) dans du méthanol (32 ml). Le milieu réactionnel est chauffé à 90°C pendant au moins 7 heures, par exemple 24 heures. Les solvants sont concentrés sous vide. L'ester éthylique d'acide 2-(6-amino-5-nitro-pyrimidin-4-yl)-2,4-dimethyl-pentanoïque (19 g; 81%) (3) est obtenu sous la forme d'un solide jaune sans plus de purification.

### c) 7-Isobutyl-7-methyl-6,7-dihydro-5H-pyrrolo[3,2-d]pyrimidin-4-amine

Une suspension de dithionite de sodium (46.78 g; 0.23 mol; 5.00 eq.) dans l'eau (136 ml) est ajoutée sur une solution chauffée à 80°C d'ester éthylique d'acide 2-(6-amino-5-nitro-pyrimidin-4-yl)-2,4-dimethyl-pentanoïque (15.74 g; 45.68 mmol; 1.00 eq.) (3) dans l'éthanol (271 ml). Le milieu réactionnel est chauffé à 80°C pendant au moins 1 heure, par exemple 5 heures. Le mélange réactionnel est ramené à température ambiante pendant la nuit puis filtré sur célite. Le filtrat est concentré sous vide pour fournir l'ester éthylique d'acide 2-(6-amino-5-sulfoamino-pyrimidin-4-yl)-2,4-dimethyl-pentanoïque (16.00 g; 101%). Le produit est directement engagé dans l'étape suivante de cyclisation.

Du chlorure d'hydrogène 4M (80.00 ml; 4.00 M; 0.32 mol; 5.00 V) dans du 1,4-dioxane est ajouté sur une suspension d'ester éthylique d'acide 2-(6-amino-5-sulfoamino-pyrimidin-4-yl)-2,4-dimethyl-pentanoïque (16.00 g; 45.68 mmol; 1.00 eq.) dans du 1,4-dioxane (320.00 ml). La suspension blanche est agitée à température ambiante pendant 30 minutes. Une partie de l'acide chlorhydrique est éliminé sous flux d'azote et le milieu réactionnel est dilué puis versé sur un mélange glace/solution d'ammoniaque 32% pour le neutraliser. Le produit est extrait à l'acétate d'éthyle/n-butanol (90/10). Les phases organiques sont rassemblées, lavées avec une solution saturée de chlorure de sodium, séchées sur sulfate de magnésium, filtrées et évaporées sous vide. Plusieurs co-évaporations à l'heptane sont réalisées pour entraîner le n-butanol. Le 4-amino-7-isobutyl-7-methyl-5,7-dihydro-6H-pyrrolo[3,2-d]pyrimidin-6-one (11.30 g; 105.07%) (4) est obtenu sous la forme d'un solide jaune pâle.

Une solution d'aluminohydrure de lithium dans du tetrahydrofurane (106.76 ml; 1.00 M; 0.11 mol; 2.20 eq.) est ajouté à 0°C au goutte à goutte sur une solution de 4-amino-7-isobutyl-7-methyl-5,7-dihydro-6*H*-pyrrolo[3,2-d]pyrimidin-6-one (11.30 g; 45.68 mmol; 1.00 eq.) (4) dans du tetrahydrofurane (267 ml). Après retour à température ambiante, le mélange réactionnel est chauffé à 90°C pendant 7 heures. Le milieu réactionnel est refroidi à 0°C. 4.2 ml d'eau, 4.2 ml d'une solution aqueuse de soude 15% (3N) puis 12.6 ml d'eau sont rajoutés goutte à goutte et l'agitation est laissée pendant 30 minutes à température ambiante puis 160 ml d'acétate d'éthyle sont rajoutés. Le sulfate de magnésium est ajouté pour sécher la phase organique et le milieu réactionnel est agité pendant 40 minutes supplémentaires. Le précipité est filtré sur célite et le filtrat est évaporé sous vide pour fournir le 7-isobutyl-7-methyl-6,7-dihydro-5H-pyrrolo[3,2-*d*]pyrimidin-4-ylamine (8.6 g; 91%) (5) sous la forme d'un solide beige.

### d) 7-isobutyl-7-methyl-5-(2-methylbenzo[d]oxazol-5-yl)-6,7-dihydro-5H-pyrrolo[3,2-d]pyrimidin-4-amine

Le 7-isobutyl-7-methyl-6,7-dihydro-5H-pyrrolo[3,2-*d*]pyrimidin-4-ylamine (1.00 g; 4.50 mmol; 1.00 eq.) (5) et 5-bromo-2-methyl-benzooxazole (1.14 g; 5.40 mmol; 1.20 eq.) sont placés dans un ballon en présence de toluène et évaporés à sec (2x) pour éliminer toutes traces d'eau. Du tert-butoxyde de sodium (1.30 g; 13.50 mmol; 3.00 eq.) puis le mélange de 2-dicyclohexylphosphino-2',6'-diisopropoxybiphenyl (RuPhos) (419.84 mg; 0.90 mmol; 0.20 eq.) et de l'adduit chloro-(2-dicyclohexylphosphino-2',6'-diisopropoxy-1,1'-biphenyl) [2-(2-aminoethyl)phenyl]palladium(II)-methyl-*t-*butyl ether (adduit RuPhos Pd G1 Methyl *t*-Butyl Ether) (734.89 mg; 0.90 mmol; 0.20 eq.) sont ajoutés sur le mélange ci-dessus en solution dans le toluène (37 ml). Le mélange réactionnel est chauffé à 100°C pendant 4 heures. Le milieu réactionnel est hydrolysé puis dilué avec de l'acétate d'éthyle. Le produit est extrait à l'acétate d'éthyle. Les phases organiques sont rassemblées, lavées une fois à l'eau, une fois avec une solution saturée de chlorure de sodium, séchées sur sulfate de magnésium, filtrées et évaporées sous vide. Le produit brut est obtenu sous la forme d'une pâte rouge-sang et chromatographié sur gel de silice avec dépôt solide (colonne puriFlash IR-50SI/300G, CombiFlash) et élution au dichlorométhane / méthanol (99/1 à 95/5). Le 7-isobutyl-7-methyl-5-(2-methyl-benzooxazol-5-yl)-6,7-dihydro-5*H*-pyrrolo[3,2-d] pyrimidin-4-ylamine (1.1 g; 70%) (6) est obtenu sous la forme d'une meringue orange foncée.

### e) 5-(4-amino-7-isobutyl-7-methyl-6,7-dihydro-5H-pyrrolo[3,2-d] pyrimidin-5-yl) benzo [d] oxazol-2-amine

Une solution d'acide chlorhydrique 35% (41.60 ml) est ajoutée sur une solution de 7-isobutyl-7-methyl-5-(2-methyl-benzooxazol-5-yl)-6,7-dihydro-5*H-*pyrrolo[3,2-d]pyrimidin-4-ylamine (2.08 g; 6.16 mmol; 1.00 eq.) (6) dans un mélange éthanol (41.60 ml) / eau (41.60 ml). Le milieu réactionnel est chauffé à 40°C pendant 4 heures. Les solvants sont ensuite éliminés sous vide pour récupérer le produit dans l'eau. Le milieu est versé sur un mélange de 300 ml glace / 30 ml d'une solution d'ammoniaque aqueux à 32%. Le produit est extrait avec un mélange acétate d'éthyle / *n*-butanol (90/10) (2x). Les phases organiques sont rassemblées, lavées une fois par une solution saturée de chlorure de sodium, séchées sur sulfate de magnésium, filtrées et évaporées sous vide. Le 2-amino-4-(4-amino-7-isobutyl-7-methyl-6,7-dihydro-5*H-*pyrrolo[3,2-*d*]pyrimidin-5-yl)-phenol (7) est obtenu sous la forme d'une meringue rouge et est directement engagé dans l'étape suivante.

Du di(1*H*-imidazol-1-yl)methanimine (2.09 g; 12.98 mmol; 1.80 eq.) est ajouté à une solution de 2-amino-4-(4-amino-7-isobutyl-7-methyl-6,7-dihydro-5*H-*pyrrolo[3,2-*d*]pyrimidin-5-yl)-phenol brut (2.26 g; 6.16 mmol; 1.00 eq.) (7) dans du tetrahydrofurane (34 ml). Le milieu réactionnel est chauffé à 70°C pendant 8 heures. Après retour à température ambiante, le milieu est versé dans de l'eau et de l'acétate d'éthyle. Les deux phases sont séparées et la phase aqueuse est extraite à l'acétate d'éthyle. Les phases organiques sont rassemblées, lavées à l'eau, séchées sur sulfate de magnésium, filtrées et évaporées. Le résidu est chromatographié sur gel de silice avec dépôt solide (colonne puriFlash IR-50SI/120G, CombiFlash) élué au dichlorométhane / méthanol (97/3). Le 5-(4-amino-7-isobutyl-7-methyl-6,7-dihydro-5*H*-pyrrolo[3,2-d]pyrimidin-5-yl)benzo[d]oxazol-2-amine (1.9 g; 90%) (8) est obtenu sous la forme d'un solide beige pour séparation par SFC des deux énantiomères. Les deux énantiomères sont obtenus avec un rendement de 36% par séparation chirale selon les conditions suivantes : Colonne : ID (Chiral Technologies) 150mm^{∗}3mm, 3µm ; température : 35°C ; pression CO₂ : 104 bars ; Co-solvant : 25% de méthanol ; débit total: 1.5 ml/min.

L'énantiomère 1 (exemple 1) de 5-(4-Amino-7-isobutyl-7-methyl-6,7-dihydro-5H-pyrrolo[3,2-d]pyrimidin-5-yl)benzo[d]oxazol-2-amine correspond au temps de rétention 2,8min.

1H RMN (DMSO-d6) δ: 0.70 (d, J = 6.4 Hz, 3H), 0.83 (d, J = 6.4 Hz, 3H), 1.19 (s, 3H), 1.30 - 1.46 (m, 1H), 1.57 - 1.72 (m, 2H), 3.73 (d, J = 10.5 Hz, 1H), 3.84 (d, J = 10.5 Hz, 1H), 5.74 (s, 2H), 6.44 (dd, J = 8.5, 2.2 Hz, 1H), 6.62 (d, J = 2.4 Hz, 1H), 7.23 (d, J = 8.4 Hz, 1H), 7.35 (s, 2H), 8.12 (s, 1H)

MS (ESI) m/z = 339 [M+H]+

L'énantiomère 2 (exemple 2) de 5-(4-Amino-7-isobutyl-7-methyl-6,7-dihydro-5*H*-pyrrolo[3,2-d]pyrimidin-5-yl)benzo[d]oxazol-2-amine correspond au temps de rétention 3,7min.

1H RMN (DMSO-d6) δ: 0.70 (d, J = 6.4 Hz, 3H), 0.83 (d, J = 6.4 Hz, 3H), 1.19 (s, 3H), 1.30 - 1.46 (m, 1H), 1.57 - 1.72 (m, 2H), 3.73 (d, J = 10.5 Hz, 1H), 3.84 (d, J = 10.5 Hz, 1H), 5.74 (s, 2H), 6.44 (dd, J = 8.5, 2.2 Hz, 1H), 6.62 (d, J = 2.4 Hz, 1H), 7.23 (d, J = 8.4 Hz, 1H), 7.35 (s, 2H), 8.12 (s, 1H)

MS (ESI) m/z = 339 [M+H]+

### Exemple 3: 5-(2-amino-benzooxazol-5-yl)-7-isobutyl-6,7-dihydro-5H-pyrrolo[3,2-d]pyrimidin-4-ylamine

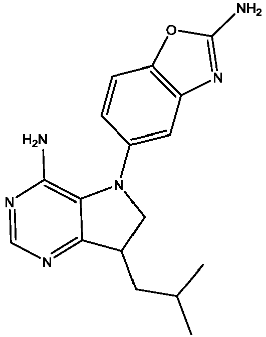

Ce composé peut être obtenu selon le procédé présenté dans la Figure 1. 1H RMN (Methanol-d4) δ: 1.00 (d, J = 8.3 Hz, 6H), 1.34 (br s, 1H), 1.90 (dd, J = 13.8, 7.1 Hz, 1H), 2.37 (br s, 2H), 3.39 (m, 2H), 7.14 (d, J = 9.2 Hz, 1H), 7.29 (br s, 2H), 7.39 (d, J = 8.8 Hz, 1H)

MS (ESI) *m*/*z* = 325 [M+H]+

### Exemple 4: 5-(2-amino-benzooxazol-5-yl)-7-methyl-7-methylsulfanylmethyl-6,7-dihydro-5H-pyrrolo[3,2-d]pyrimidin-4-ylamine

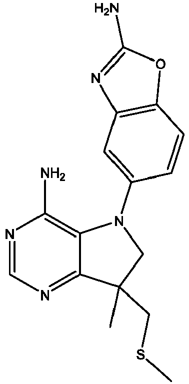

Ce composé peut être obtenu selon le procédé présenté dans la Figure 1. 1H RMN (Methanol-d4) δ: 1.39 (s, 3H), 1.99 (s, 3H), 2.76 - 2.93 (m, 2H), 3.78 (d, J = 10.4 Hz, 1H), 4.09 (d, J = 10.5 Hz, 1H), 6.67 (d, J = 8.6 Hz, 1H), 6.82 (s, 1H), 7.23 (d, J = 8.3 Hz, 1H), 8.12 (s, 1H)

MS (ESI) *m*/*z* = 343 [M+H]+

### Exemple 5 : voie de synthèse du composé 5-(4-amino-7,7-diethyl-6,7-dihydro-5H-pyrrolo [3,2-d] pyrimidin-5-yl)benzo[d]oxazol-2-amine

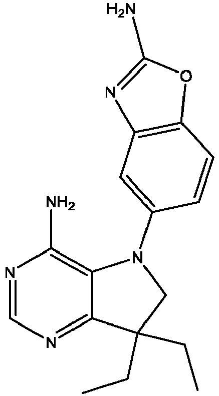

### a) Ethyl-2-ethyl-2-(6-methoxy-5-nitropyrimidin-4-yl)butanoate

Du iodoéthane (399.97 µl, 4.98 mmol; 1.20 eq.) est ajouté sur une suspension d'ester éthylique d'acide (6-methoxy-5-nitro-pyrimidin-4-yl)-acétique (1.00 g; 4.15 mmol; 1.00 eq.) et de carbonate de césium (1.42 g; 4.35 mmol; 1.05 eq.) dans l'acétonitrile (15 ml). Le milieu réactionnel est chauffé à 70°C pendant 12 heures. Du carbonate de césium (1.62 g; 4.98 mmol; 1.20 eq.) suivi de iodoéthane (499.96 µl, 6.22 mmol; 1.50 eq.) sont ajoutés et le milieu réactionnel est chauffé à 70°C pendant 5 heures. Après retour à température ambiante, le milieu réactionnel est filtré pour éliminer le carbonate de césium puis le filtrat est concentré sous vide. Le résidu est chromatographié sur gel de silice (40g, dépôt liquide, éluant heptane/acétate d'éthyle de 0 à 5% d'acétate d'éthyle, CCM : heptane/acétate d'éthyle 50/50 Rf=0.8) pour fournir le ethyl-2-ethyl-2-(6-methoxy-5-nitropyrimidin-4-yl)butanoate (1.0 g; 81%) sous la forme d'une huile claire.

### b) Ethyl-2-(6-amino-5-nitropyrimidin-4-yl)-2,4-dimethylpentanoate

Dans un tube micro-onde scellé, de l'ammoniaque 7N dans du méthanol (3.0 ml) est ajouté sur une solution de ethyl-2-ethyl-2-(6-methoxy-5-nitropyrimidin-4-yl)butanoate (1.00 g; 3.36 mmol; 1.00 eq.) dans du méthanol (5.0 ml). Le milieu réactionnel est chauffé à 70°C pendant 12 heures. Les solvants sont concentrés sous vide. L'ethyl-2-(6-amino-5-nitropyrimidin-4-yl)-2-ethylbutanoate (950 mg) est obtenu sous la forme d'un solide jaune sans plus de purification.

### c) 4-amino-7,7-diethyl-5,7-dihydro-6H-pyrrolo[3,2-d]pyrimidin-6-one

Une suspension d'ethyl 2-(6-amino-5-nitropyrimidin-4-yl)-2-ethylbutanoate (0.95 g; 3.37 mmol; 1.00 eq.) dans l'éthanol (38.00 ml) est additionné sur une solution chauffée à 80°C de dithionite de sodium (2.93 g; 16.83 mmol; 5.00 eq.) dans l'eau (9.50 ml). Le milieu réactionnel est chauffé à 80°C pendant 1h15. Le mélange réactionnel est ramené à température ambiante puis filtré pour éliminer les insolubles. Le filtrat est concentré sous vide pour donner l'acide 4-amino-6-(3-(ethoxycarbonyl)pentan-3-yl)pyrimidin-5-yl)sulfamique (1.12 g; 100.13%) sous la forme d'un solide blanc (présence de sels). Le produit est directement engagé dans l'étape de cyclisation en considérant un rendement quantitatif.

Du chlorure d'hydrogène 4M dans du 1,4-dioxane (11.2 ml) est ajouté sur une suspension d'acide (4-amino-6-(3-(ethoxycarbonyl)pentan-3-yl)pyrimidin-5-yl)sulfamique (1.12 g; 3.37 mmol; 1.00 eq.) dans du 1,4-dioxane (34 ml). La suspension blanche est agitée à température ambiante pendant 30 minutes. Une partie de l'acide chlorhydrique est éliminé sous flux d'azote et le milieu réactionnel est dilué puis versé sur un mélange glace/solution d'ammoniaque 32% pour le neutraliser. Le produit est extrait à l'acétate d'éthyle / n-butanol (90/10). Les phases organiques sont rassemblées, lavées avec une solution saturée de chlorure de sodium, séchées sur sulfate de magnésium, filtrées et évaporées sous vide. Plusieurs co-évaporations à l'heptane sont réalisées pour entraîner le n-butanol. Le 4-amino-7,7-diethyl-5,7-dihydro-6H-pyrrolo[3,2-d]pyrimidin-6-one (690 mg; 99%) est obtenu sous la forme d'un solide jaune pâle.

### d) 7,7-diethyl-6,7-dihydro-5H-pyrrolo[3,2-d]pyrimidin-4-amine

Une solution d'aluminohydrure de lithium dans du tetrahydrofurane (7.36 ml; 1.00 M; 7.36 mmol; 2.20 eq.) est ajouté à 0°C au goutte à goutte sur une solution de 4-amino-7,7-diethyl-5,7-dihydro-6H-pyrrolo[3,2-d]pyrimidin-6-one (690.00 mg; 3.35 mmol; 1.00 eq.) dans du tetrahydrofurane (18 ml). Après retour à température ambiante, le mélange réactionnel est chauffé à 90°C pendant 7 heures. Le milieu réactionnel est refroidi à 0°C. 0.3 ml d'eau, 0.3 ml d'une solution aqueuse de soude 15% (3N) puis 0.9 ml d'eau sont rajoutés goutte à goutte et l'agitation est laissée pendant 30 minutes à température ambiante puis 5 ml d'acétate d'éthyle sont rajoutés. Le sulfate de magnésium est ajouté pour sécher la phase organique et le milieu réactionnel est agité pendant 40 minutes supplémentaires. Le précipité est filtré sur celite et le filtrat est évaporé sous vide. Le brut réactionnel est purifié en phase normale (25g, dépôt liquide, éluant dichlorométhane/méthanol de 3 à 7% de méthanol, CCM : dichlorométhane/méthanol 95/5 Rf=0.2) pour fournir le 7,7-diethyl-6,7-dihydro-5H-pyrrolo[3,2-d]pyrimidin-4-amine (480 mg; 75 %) sous la forme d'un solide beige.

### e) 7,7-diethyl-5-(2-methylbenzo[d]oxazol-5-yl)-6,7-dihydro-5H-pyrrolo[3,2-d]pyrimidin-4-amine

Le 7,7-diethyl-6,7-dihydro-5*H*-pyrrolo[3,2-*d*]pyrimidin-4-amine (200.00 mg; 1.04 mmol; 1.00 eq.) et 5-bromo-2-methyl-benzooxazole (242.64 mg; 1.14 mmol; 1.10 eq.) sont placés dans un ballon en présence de toluène et évaporés à sec (2x) pour éliminer toutes traces d'eau. Du tert-butoxyde de sodium (242.64 mg; 1.14 mmol; 1.10 eq.) puis le mélange de 2-dicyclohexylphosphino-2',6'-diisopropoxybiphenyl (RuPhos) (127.45 mg; 0.16 mmol; 0.15 eq.) et de l'adduit chloro-(2-dicyclohexylphosphino-2',6'-diisopropoxy-1,1'-biphenyl)[2-(2-aminoethyl)phenyl]palladium(II)-methyl-*t*-butyl ether (adduit RuPhos Pd G1 Methyl t-Butyl Ether) (127.45 mg; 0.16 mmol; 0.15 eq.) sont ajoutés sur le mélange ci-dessus en solution dans le toluène (8 ml). Le mélange réactionnel est chauffé à 100°C pendant 4 heures. Le milieu réactionnel est hydrolysé puis dilué avec de l'acétate d'éthyle. Le produit est extrait à l'acétate d'éthyle. Les phases organiques sont rassemblées, lavées une fois à l'eau, une fois avec une solution saturée de chlorure de sodium, séchées sur sulfate de magnésium, filtrées et évaporées sous vide. Le produit brut est obtenu sous la forme d'une pâte et chromatographié sur gel de silice avec dépôt solide et élution au dichlorométhane / méthanol (99/1 à 95/5). Le 7,7-diethyl-5-(2-methylbenzo[*d*]oxazol-5-yl)-6,7-dihydro-5*H*-pyrrolo[3,2-d] pyrimidin-4-amine (28 mg; 8.32 %) est obtenu sous la forme d'un solide rose.

### f) 2-amino-4-(4-amino-7,7-diethyl-6,7-dihydro-5H-pyrrolo[3,2-d]pyrimidin-5-yl)phenol

Une solution d'acide chlorhydrique 35% (0.56 mL) est additionnée sur du 7,7-diethyl-5-(2-methyl-benzooxazol-5-yl)-6,7-dihydro-5H-pyrrolo[3,2-d] pyrimidin-4-ylamine (28.00 mg; 0.09 mmol; 1.00 eq.) en solution dans l'éthanol (1.12 ml) et l'eau (1.12 ml). Le milieu réactionnel est chauffé à 70°C pendant 5 heures. Les solvants sont éliminés sous vide pour récupérer le produit dans l'eau. Le milieu réactionnel est versé sur une solution à 0°C de NHsaq 15% puis le pH est ajusté jusqu'à pH=8 avec une solution de NHsaq. 32%. Le produit est extrait par le mélange dichlorométhane/n-butanol (80/20) (2x). Les phases organiques sont rassemblées, lavées une fois par de l'eau puis séchées sur sulfate de magnésium, filtrées et concentrées sous vide. Le 2-amino-4-(4-amino-7,7-diethyl-6,7-dihydro-5H-pyrrolo[3,2-d]pyrimidin-5-yl)phenol (26.00 mg; 100%) est obtenu sous la forme d'un solide rose et directement engagé dans l'étape suivante.

### g) 5-(4-Amino-7,7-diethyl-6,7-dihydro-5H-pyrrolo[3,2-d]pyrimidin-5-yl)benzo[d]oxazol-2-amine

Le di(1H-imidazol-1-yl)methanimine (35.0 mg; 0.22 mmol; 2.50 eq.) est ajouté à une solution de 2-amino-4-(4-amino-7,7-diethyl-6,7-dihydro-5*H*-pyrrolo[3,2-d]pyrimidin-5-yl)phenol brut (26.0 mg; 0.09 mmol; 1.00 eq.) dans du tetrahydrofurane (1.6 ml). Le milieu réactionnel est chauffé à 70°C pendant 8 heures. Après retour à température ambiante, le milieu est versé dans de l'eau et de l'acétate d'éthyle. Les deux phases sont séparées et la phase aqueuse est extraite à l'acétate d'éthyle. Les phases organiques sont rassemblées, lavées à l'eau, séchées sur sulfate de magnésium, filtrées et évaporées. Le résidu est chromatographié sur gel de silice avec dépôt solide et élué au dichlorométhane / méthanol (99/1 à 94/6). Le 5-(4-amino-7,7-diethyl-6,7-dihydro-5H-pyrrolo[3,2-d]pyrimidin-5-yl)benzo[d]oxazol-2-amine (10 mg; 32 %) est obtenu sous la forme d'un solide blanc.

1H RMN (DMSO-d6) δ: 0.69 (t, J = 7.4 Hz, 7H), 1.49 - 1.67 (m, 5H), 3,75 (s, 1H), 5.70 (s, 2H), 6.42 (dd, J = 8.5, 2.3 Hz, 1H), 6.60 (d, J = 2.4 Hz, 1H), 7.23 (d, J = 8.5 Hz, 1H), 7.34 (s, 2H), 8.12 (s, 1H)

MS (ESI) m/z = 325 [M+H]+

### Exemple 6: 5-(2-amino-benzooxazol-5-yl)-7,7-dimethyl-6,7-dihydro-5H-pyrrolo[3,2-d]pyrimidin-4-ylamine

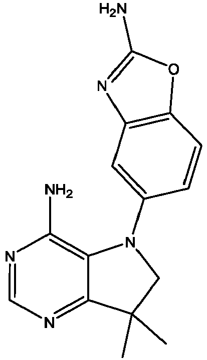

Ce composé peut être obtenu selon le procédé présenté dans la Figure 1. 1H RMN (DMSO-d6) δ: 1.18 (s, 6H), 3.71 (s, 2H), 5.79 (s, 2H), 6.43 (dd, J = 8.4, 2.3 Hz, 1H), 6.61 (d, J = 2.3 Hz, 1H), 7.22 (d, J = 8.4 Hz, 1H), 7.36 (s, 2H), 8.13 (s, 1H) MS (ESI) m/z = 297 [M+H]+

### Exemple 7: 5-(2-amino-benzooxazol-5-yl)-7-methyl-7-propyl-6,7-dihydro-5H-pyrrolo[3,2-d]pyrimidin-4-ylamine

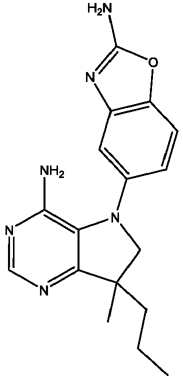

Ce composé peut être obtenu selon le procédé présenté dans la Figure 1. 1H RMN (DMSO-d6) δ: 0.78 (t, J = 7.2 Hz, 3H), 1.20 (s, 3H), 1.22 - 1.32 (m, 2H) 1.35 - 1.45 (m, 1H) 1.43 - 1.57 (m, 1H), 3.69 (d, J = 10.7 Hz, 1H), 3.79 (d, J = 10.7 Hz, 1H), 5.73 (s, 2H), 6.42 (dd, J = 8.6, 2.1 Hz, 1H), 6.61 (d, J = 2.3 Hz, 1H), 7.23 (d, J = 8.3 Hz, 1H), 7.35 (s, 2H), 8.12 (s, 1H)

MS (ESI) m/z = 325 [M+H]+

### Exemple 8: 5-(2-amino-benzooxazol-5-yl)-7,7-di ethyl-6-methyl-6,7-dihydro-5H-pyrro lo[3,2-d]pyrimidin-4-ylamine

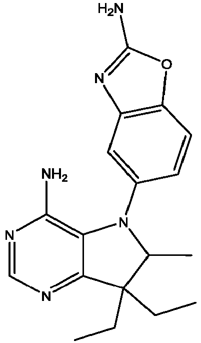

Ce composé peut être obtenu selon le procédé présenté dans la Figure 1. 1H RMN (DMSO-d6) δ: 0.77 (t, J = 7.4 Hz, 3H), 0.84 (t, J = 7.4 Hz, 3H), 1.21 (d, J = 6.6 Hz, 3H), 1.49 (m, 2H), 1.73 (m, 2H), 3.61 (m, 1H), 5.36 (s, 2H), 6.53 - 6.64 (m, 1H), 6.76 (d, J = 2.2 Hz, 1H), 7.27 (d, J = 8.4 Hz, 1H), 7.42 (s, 2H), 8.09 (s, 1H)

MS (ESI) m/z = 339 [M+H]+

### Exemple 9: 4-amino-5-(2-amino-benzooxazol-5-yl)-7,7-dimethyl-5,7-dihydro-pyrrolo[3,2-d]pyrimidin-6-one

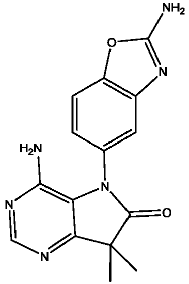

Ce composé peut être obtenu selon le procédé présenté dans la Figure 1. 1H RMN (DMSO-d6) δ: 1.35 (s, 6H), 5.44 (s, 2H), 7.01 (dd, J = 8.3, 2.2 Hz, 1H), 7.27 (d, J = 2.1 Hz, 1H), 7.45 (d, J = 8.3 Hz, 1H), 7.62 (s, 2H), 8.25 (s, 1H)

MS (ESI) m/z = 311 [M+H]+

### Exemples 10, 11 et 12: voie de synthèse des composés 1-acetyl-4'-amino-5'-(2-aminobenzo[d]oxazol-5-yl)spiro[azetidine-3,7'-cyclopenta[d]pyrimidin]-6'(5'H)-one (15 - exemple 10), tert-butyl-4'-amino-5'-(2-aminobenzo[d]oxazol-5-yl)-6'-oxo-5',6'-dihydrospiro[azetidine-3,7'-pyrrolo[3,2-d]pyrimidine]-1-carboxylate (14 - exemple 11), et tert-butyl-4'-amino-5'-(2-aminobenzo[d]oxazol-5-yl)-5',6'-dihydrospiro[azetidine-3,7'-pyrrolo[3,2-d]pyrimidine]-1-carboxylate (16 - exemple 12) telle qu'illustrée par la Figure 2

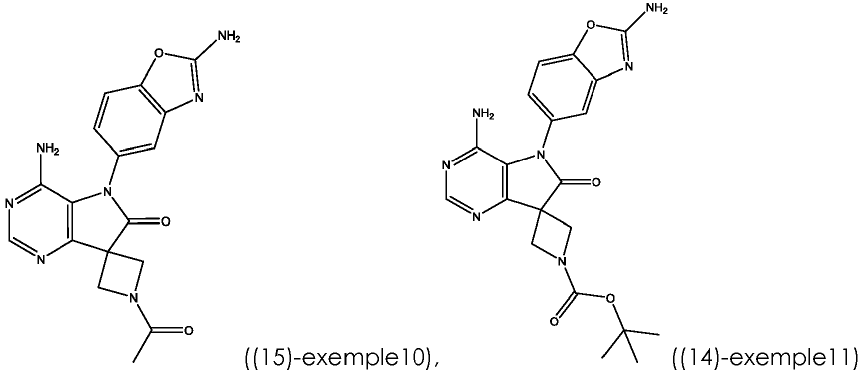

et

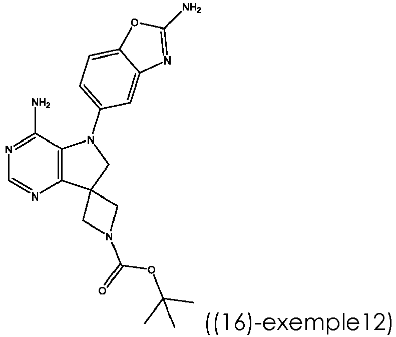

### a) 5-lodo-1,3-benzoxazol-2-amine

Dans un tube scellé, du 5-bromo-1,3-benzoxazol-2-amine (1.0 g, 4.69 mmol), iodure de sodium (1.77g, 11.73mmol), iodure de cuivre (I) (225 mg, 1.17 mmol) et *N*,*N*'-dimethylethylenediamine (414 mg, 4.69 mmol) sont agités dans 30 ml de dioxane à 120°C pendant la nuit. Du iodure de cuivre (I) (224 mg) et N,N'-dimethylethylenediamine (0.25 ml) sont ajoutés et l'agitation est poursuivie pendant 24h à 120°C. Après refroidissement, 100 ml d'EtOAc sont ajoutés et le milieu est filtré. Le filtrat est complété de 100 ml d'eau et transféré dans une ampoule à décanter. Les phases sont séparées et la phase aqueuse est extraite deux fois à l'EtOAc. Les fractions organiques rassemblées sont lavées à l'eau, à la saumure, séchées sur N₀₂SO₄ et évaporées à sec. Le résidu solide est purifié par chromatographie sur gel de silice en éluant au DCM pour obtenir le 5-iodo-1,3-benzoxazol-2-amine (318 mg, 25%).

### b) 4-chloro-6-methoxy-2-methylsulfanyl-5-nitro-pyrimidine (9)

Du 4,6-dichloro-2-methylsulfanyl-5-nitro-pyrimidine (10.0 g, 41.6 mmol) dans 102 ml de méthanol sont ajoutés lentement (9.49 ml, 41.6 mmol) de méthylate de sodium 30% en poids (un précipité jaune pâle se forme). Le milieu réactionnel est refroidi à 0°C, le précipité est filtré puis rincé au méthanol deux fois et séché à l'étuve à vide à 40°C pour obtenir 4.62g d'une poudre jaune pâle. Le filtrat est concentré à sec puis resuspendu dans l'eau, filtré et séché à l'étuve à vide à 40°C pour obtenir 5.5g supplémentaires d'une poudre jaune pâle. Les deux fractions obtenues contiennent 70% de produit attendu (4-chloro-6-methoxy-2-methylsulfanyl-5-nitro-pyrimidine - (9)) et 30% de l'analogue diméthoxy.

### c) 1-(tert-butyl)-3-methyl-3-(6-methoxy-2-(methylthio)-5-nitropyrimidin-4-yl)azetidine-1,3-dicarboxylate (10)

A une solution de 4-chloro-6-methoxy-2-methylsulfanyl-5-nitro-pyrimidine (4.27 g, 17.2 mmol, 1.0 eq.) (9) dans du tetrahydrofurane (86 ml), sont ajoutés du 1-(*tert*-butyl)-3-methylazetidine-1,3-dicarboxylate (3.56 ml, 18.08 mmol, 1.1 eq.). Le mélange hétérogène résultant a été refroidi à -78°C, puis du LiHMDS 1M dans du THF (18.07 ml, 18.08 mmol, 1.1 eq.) a été ajouté goutte à goutte et le mélange réactionnel a été agité à température ambiante pendant 22 heures. Le mélange réactionnel a été versé lentement dans de l'eau et de l'acétate d'éthyle. Les couches ont été séparées et la couche aqueuse a été soumise à une réextraction avec de l'acétate d'éthyle. Les couches organiques combinées ont été lavées avec de l'eau et de la saumure, séchées sur Na₂SO₄ et concentrées sous pression réduite. Le produit brut a été purifié par chromatographie éclair sur gel de silice en utilisant du dichlorométhane dans l'heptane de 50% à 100% pour donner le composé souhaité (1-(*tert*-butyl)-3-methyl-3-(6-methoxy-2-(methylthio)-5-nitropyrimidin-4-yl)azetidine-1,3-dicarboxylate (10)) sous la forme d'une poudre jaune (4.73 g, rendement 66%).

### d) 1-(tert-butyl)-3-methyl-3-(6-amino-2-(methylthio)-5-nitropyrimidin-4-yl)azetidine-1,3-dicarboxylate (11)

Du 1-(*tert*-butyl)-3-methyl-3-(6-methoxy-2-(methylthio)-5-nitropyrimidin-4-yl)azetidine-1,3-dicarboxylate (4.71 g, 11.36 mmol, 1.0 eq.) (10) a été dilué dans une solution d'ammoniaque 7 N dans du méthanol (38 ml, 266 mmol, 20.0 eq.). La suspension résultante a été agitée pendant 3h30 à température ambiante. Ensuite, le précipité formé a été filtré et lavé avec du méthanol pour donner le composé attendu (1-(*tert*-butyl)-3-methyl-3-(6-amino-2-(methylthio)-5-nitropyrimidin-4-yl)azetidine-1,3-dicarboxylate (11)) sous la forme d'une poudre blanche (3.75 g, rendement 78%).

### e) Tert-butyl-4'-amino-2'-(methylthio)-6'-oxo-5',6'-dihydrospiro[azetidine-3,7'-pyrrolo[3,2-d]pyrimidine]-1-carboxylate (12)

A une suspension de 1-(*tert*-butyl)-3-methyl-3-(6-amino-2-(methylthio)-5-nitropyrimidin-4-yl)azetidine-1,3-dicarboxylate (3.53 g, 8.84 mmol, 1.0 eq.) (11) dans du méthanol (44 ml), de la poudre de fer (2.47 g, 44.2mmol, 5.0 eq.) a été ajoutée, suivie par du chlorure d'ammonium (2.36 g, 44.2 mmol, 5.0 eq.). Le mélange hétérogène résultant a été chauffé au reflux pendant 3h30. La solution a été refroidie à température ambiante et agitée à cette température pendant une nuit. Le mélange a ensuite été filtré sur de la poudre de talc et rincé plusieurs fois avec du méthanol. Le filtrat a été concentré à sec et précipité dans l'eau pour donner le composé attendu (*Tert*-butyl-4'-a mino-2'-(methylthio)-6'-oxo-5',6'-dihydrospiro[azetidine-3,7'-pyrrolo[3,2-*d*]pyrimidine]-1-carboxylate (12)) sous la forme d'une poudre beige (2.35 g, rendement 79%).

### f) Tert-butyl-4'-amino-5'-(2-aminobenzo[d]oxazol-5-yl)-2'-(methylthio)-6'-oxo-5',6'-dihydrospiro[azetidine-3,7'-pyrrolo[3,2-d]pyrimidine]-1-carboxylate (13)

A une solution de *tert*-butyl-4-amino-2-methylsulfanyl-6-oxo-spiro[5H-pyrrolo[3,2-*d*]pyrimidine-7,3'-azetidine]-1'-carboxylate (500.0 mg, 1.48 mmol, 1.0 eq.) dans 10 ml de DMF sec (10 ml), du tert-butoxyde de potassium (532 mg, 4.74 mmol, 3.0 eq.) a été ajouté. Après 5 min d'agitation, on ajoute au mélange réactionnel de la 5-iodo-1,3-benzoxazol-2-amine (1.16 g, 4.45 mmol, 3.0 eq.), puis de la N, N-diméthylglycine (519.9 mg, 4.89 mmol. 3.0 eq.) et du bromure de cuivre (I) (235 mg, 1.63 mmol, 1.0 eq.). Le mélange réactionnel a été dégazé avec de l'argon, puis le tube a été scellé et chauffé à 145°C pendant 7 heures. Ensuite, une solution de KHSO₄ 1M a été ajoutée jusqu'à pH = 5 et le mélange a été extrait avec de l'acétate d'éthyle. Les couches ont été séparées et la couche aqueuse a ensuite été soumise à une réextraction avec de l'acétate d'éthyle. Les couches organiques combinées ont été lavées avec de l'eau et de la saumure, séchées sur N₀₂SO₄ et concentrées sous pression réduite. Le produit brut a été purifié par chromatographie éclair sur gel de silice en utilisant du méthanol dans du dichlorométhane de 0% à 10% pour obtenir le *tert*-butyl-4'-amino-5'-(2-aminobenzo[*d*]oxazol-5-yl)-2'-(methylthio)-6'-oxo-5',6'-dihydrospiro[azetidine-3,7'-pyrrolo[3,2-d] pyrimidine]-1-carboxylate (148 mg, rendement 21%) (13).

### g) Tert-butyl-4'-amino-5'-(2-aminobenzo[d]oxazol-5-yl)-6'-oxo-5',6'-dihydrospiro[azetidine-3,7'-pyrrolo[3,2-d]pyrimidine]-1-carboxylate (14 - exemple 11)

Une solution de *tert*-butyl-4'-amino-5'-(2-aminobenzo[*d*]oxazol-5-yl)-2'-(methylthio)-6'-oxo-5',6'-dihydrospiro[azetidine-3,7'-pyrrolo[3,2-d] pyrimidine]-1-carboxylate (75.0 mg, 0.16 mmol, 1.00 eq.) (13) dans du 1,4-dioxane/MeOH (1:1, 10 ml) a été hydrogéné sur un appareil H-CUBE PRO en utilisant une cartouche Ni-Raney (0.5 ml/min, 7 bar, 50°C). Le mélange réactionnel a été évaporé pour donner le composé attendu (tert-butyl-4'-amino-5'-(2-aminobenzo[*d*]oxazol-5-yl)-6'-oxo-5',6'-dihydrospiro[azetidine-3,7'-pyrrolo[3,2-*d*]pyrimidine]-1-carboxylate (14-exemple 11)) (50 mg, rendement 74%).

1H RMN (DMSO-d6) δ: 1.43 (s, 9H), 4.08 (b, 4H), 5.53 (b, 2H), 7.01 (dd, J = 2.05, 8.30 Hz, 1H), 7.26 (d, J = 2.05 Hz, 1H), 7.45 (d, J = 8.30 Hz, 1H), 7.60 (s, 2H), 8.33 (s, 1H).

MS (ESI) *m*/*z* = 424 [M+H]+

### h) 1-acetyl-4'-amino-5'-(2-aminobenzo[d]oxazol-5-yl)spiro[azetidine-3,7'-cyclopenta[d]pyrimidin]-6'(5'H)-one (15-exemple 10)

A une solution de tert-butyl-4'-amino-5'-(2-aminobenzo[d]oxazol-5-yl)-6'-oxo-5',6'-dihydrospiro[azetidine-3,7'-pyrrolo[3,2-d] pyrimidine]-1-carboxylate (48.0 mg, 0.11 mmol, 1.0 eq.) (14 - exemple 11) dans du DCM (1 ml) à 4°C sous N₂, de l'acide trifluoroacétique (0.5 ml) a été ajouté goutte à goutte. Après 4 heures, le mélange réactionnel a été concentré sous vide. Le résidu a été dissous dans du DCM / MeOH (9:1) et passé à travers une lame d'alumine basique en utilisant un mélange DCM / MeOH (9:1) comme éluant. Les fractions contenant le composé attendu ont été évaporées et le produit brut directement engagé dans l'étape suivante.

A une solution de 4'-amino-5'-(2-amino-1,3-benzoxazol-5-yl)spiro[azetidine-3,7'-pyrrolo[3,2-d]pyrimidine]-6'-one (38.0 mg, 0.12 mmol, 1.0 eq.) dans du DCM (2 ml) refroidi à -78°C sous N₂, de la triéthylamine (18.2 µl, 0.13 mmol, 1.1 eq.) et du chlorure d'acétyle (8,43 µL, 0,12 mmol, 1,0 eq.) ont été respectivement ajoutés. Le mélange réactionnel a été maintenu à -78°C pendant 2 heures, suivi d'un retour lent à température ambiante. Une solution d'eau froide (4°C) a ensuite été ajoutée et le mélange biphasique a été agité pendant 5 minutes. Il a ensuite été transféré dans un entonnoir de séparation, la couche organique a été recueillie et la phase aqueuse a été extraite deux fois avec du DCM. Les couches organiques combinées ont été séchées sur du sulfate de sodium, filtrées et séchées sous vide. Le résidu solide a été purifié par chromatographie sur une colonne d'alumine basique en utilisant un gradient de DCM / MeOH de 98:2 à 9:1. Les fractions pures ont été combinées pour donner le composé attendu (1-acetyl-4'-amino-5'-(2-aminobenzo[*d*]oxazol-5-yl)spiro[azetidine-3,7'-cyclopenta[*d*]pyrimidin]-6'(5'*H*)-one (15 - exemple 10)) sous la forme d'une poudre blanche (2.2 mg, rendement 5%).

1H RMN (DMSO-d6) δ: 1.86 (s, 3H), 4.09 (b, 2H), 4.38 (b, 2H), 5.51 (b, 2H), 7.03 (dd, J = 2.12, 8.20 Hz, 1H), 7.27 (d, J = 2.12 Hz, 1H), 7.45 (d, J = 8.20 Hz, 1H), 7.61 (s, 2H), 8.33 (s, 1H).

MS (ESI) *m*/*z* = 366 [M+H]+

### h') Tert-butyl-4'-amino-5'-(2-aminobenzo[d]oxazol-5-yl)-2'-(methylthio)-5',6'-dihydrospiro[azetidine-3,7'-pyrrolo[3,2-d]pyrimidine]-1-carboxylate

A une solution de *tert*-butyl-4'-amino-5'-(2-amino-1,3-benzoxazol-5-yl)-2'-methylsulfanyl-6'-oxo-spiro[azetidine-3,7'-pyrrolo[3,2-*d*] pyrimidine]-1-carboxylate (145.0 mg, 0.31 mmol, 1.0 eq.) dans 3 ml de tetrahydrofurane, une solution du complexe borane-méthylsulfure 2M dans du dichlorométhane (1.39 ml, 2.78 mmol, 1.0 eq.) a été ajoutée. Le mélange réactionnel a été agité à température ambiante pendant une nuit et 1 heure à 60°C. Ensuite, de l'eau et de l'acétate d'éthyle ont été ajoutés. Les couches ont été séparées et la couche aqueuse a ensuite été soumise à une réextraction avec de l'acétate d'éthyle. Les couches organiques combinées ont été lavées avec de l'eau et de la saumure, séchées sur N₀₂SO₄ et concentrées sous pression réduite. Le produit brut a été purifié par chromatographie éclair sur gel de silice en utilisant du méthanol dans du dichlorométhane de 2% à 15% pour obtenir le composé désiré tert-butyl-4'-amino-5'-(2-aminobenzo[d]oxazol-5-yl)-2'-(methylthio)-5',6'-dihydrospiro [azetidine-3,7'-pyrrolo [3,2-d] pyri midine]-1-carboxylate (26 mg, rendement 19%).

### i') Tert-butyl-4'-amino-5'-(2-aminobenzo[d]oxazol-5-yl)-5',6'-dihydrospiro[azetidine-3,7'-pyrrolo[3,2-d]pyrimidine]-1-carboxylate (16 - exemple 12)

Du tert-butyl-4'-amino-5'-(2-aminobenzo[d]oxazol-5-yl)-2'-(methylthio)-6'-oxo-5',6'-dihydrospiro[azetidine-3,7'-pyrrolo[3,2-d]pyrimidine]-1-carboxylate (10.0 mg, 0.022 mmol, 1.0 eq.) a été dissous dans 4 ml d'un mélange de 1,4-dioxane et de méthanol 1:1. La solution résultante a été filtrée sur millipore et hydrogénée en présence de nickel de Raney sur un appareil H-CUBE PRO (0,5 ml/mn, 7 bars, 50°C). Le système a été rincé avec 20 ml de DMSO pour donner une solution contenant le composé attendu. Le volume a été réduit sous pression réduite et l'huile ainsi obtenue a ensuite été purifiée par chromatographie sur RP18 phase inverse en utilisant un gradient de méthanol dans l'eau de 30% à 50%, pour donner le composé souhaité (*tert*-butyl-4'-amino-5'-(2-aminobenzo[*d*] oxazol-5-yl)-5',6'-dihydrospiro[azetidine-3,7'-pyrrolo[3,2-d]pyrimidine]-1-carboxylate (16 - exemple 12)) (0.8 mg, rendement 10%).

1H RMN (DMSO-d6) δ: 1.46 (s, 9H), 4.04 (b, 2H), 4.20 (s, 2H), 4.26 (b, 2H), 6.71 (dd, *J* = 2.07, 8.41 Hz, 1H), 6.86 (d, *J* = 2.07 Hz, 1H), 7.25 (d, *J* = 8.41 Hz, 1H), 8.19 (s, 1H).

MS (ESI) *m*/*z* =410 [M+H]+

### Exemple 13: activités enzymatique mTOR et cellulaire mTORCl/mTORC2

### 13.1 Activité inhibitrice de la kinase mTOR

Le modèle de criblage de l'activité inhibitrice des molécules sur mTOR a été développé avec la technologie LANTHASCREEN^{™} (Lifetechnologies). Le substrat de la réaction (400 nM final), les dilutions sérielles des molécules (1% DMSO final) et l'enzyme (<1 nM) sont ajoutés successivement dans une plaque 384 puits (Corning 4514) dans un volume final de 10 pL par puits. Après 1 heure de réaction à température ambiante, 10 µL d'une solution contenant 10 mM final d'EDTA et 2 nM final d'anticorps marqués au Terbium sont ajoutés. Après au moins 30 minutes d'incubation à température ambiante, le signal TR-FRET est mesuré avec un lecteur de microplaque adapté selon les recommandations du fournisseur. Les données sont normalisées par des contrôles positifs ('POS' contenant une concentration saturante d'inhibiteur de référence) et des contrôles négatifs ('NEG' contenant 1% DMSO) : % inhibition = ((X-NEG)^{∗}100)/(POS-NEG). Les IC50 sont calculées à partir d'un modèle logistique à 4 paramètres à l'aide du logiciel XLFit (IDBS).

### 13.2 Activité inhibitrice mTORC1/mTORC2

Les cellules A431 sont ensemencées en milieu complet (DMEM+10% SVF) à 25 000 cellules par puits d'une plaque 96 puits coatés à la poly-L-Lysine. 24 heures avant l'expérience, le milieu est remplacé par du milieu sans sérum. Les dilutions sérielles des molécules à tester sont ajoutées (0.1% DMSO final). Après 3 heures d'incubation à 37°C, la phosphorylation des biomarqueurs S6RP (mTORC1) et AKT (mTORC2) est mesurée à l'aide de la technologie HTRF (Cisbio) selon les recommandations du fournisseur. Les données sont normalisées par des contrôles positifs ('POS' contenant une concentration saturante d'inhibiteur de référence) et des contrôles négatifs ('NEG' contenant 1% DMSO) : % inhibition=((X-NEG)^{∗}100)/(POS-NEG). Les IC50 sont calculées à partir d'un modèle logistique à 4 paramètres à l'aide du logiciel XLFit (IDBS).

Tableau des résultats d'activités :

| Exemple | Nom chimique | Ki mTOR (nM) | IC50 mTORC1 (nM) | IC50 mTORC2 (nM) |
|---|---|---|---|---|
| 1 | 5-(2-amino-benzooxazol-5-yl)-7-isobutyl-7-methyl-6,7-dihydro-5H-pyrrolo[3,2-d]pyrimidin-4-ylamine | 2.0 | 1.0 | 2.2 |
| 2 | 5-(2-amino-benzooxazol-5-yl)-7-isobutyl-7-methyl-6,7-dihydro-5H-pyrrolo[3,2-d]pyrimidin-4-ylamine | 130.0 | 68.5 | 136.8 |
| 3 | 5-(2-amino-benzooxazol-5-yl)-7-isobutyl-6,7-dihydro-5H-pyrrolo[3,2-d]pyrimidin-4-ylamine | 11.7 | 1.8 | 11.4 |
| 4 (racémique) | 5-(2-amino-benzooxazol-5-yl)-7-methyl-7-methylsulfanylmethyl-6,7-dihydro-5H-pyrrolo[3,2-d]pyrimidin-4-ylamine | 3.6 | 11.7 | 14.5 |
| 5 | 5-(2-amino-benzooxazol-5-yl)-7,7-diethyl-6,7-dihydro-5H-pyrrolo[3,2-d]pyrimidin-4-ylamine | 34.6 | 8.6 | 16.3 |
| 6 | 5-(2-amino-benzooxazol-5-yl)-7,7-dimethyl-6,7-dihydro-5H-pyrrolo[3,2-d]pyrimidin-4-ylamine | 37.8 | 17.7 | 31.4 |
| 7 | 5-(2-amino-benzooxazol-5-yl)-7-methyl-7-propyl-6,7-dihydro-5H-pyrrolo[3,2-d]pyrimidin-4-ylamine | 8.7 | 19.5 | 34.5 |
| 8 | 5-(2-amino-benzooxazol-5-yl)-7,7-di ethyl-6-methyl-6,7-dihydro-5H-pyrrolo[3,2-d]pyrimidin-4-ylamine | 212.7 | 151.6 | 242.8 |
| 9 | 4-amino-5-(2-amino-benzooxazol-5-yl)-7,7-dimethyl-5,7-dihydro-pyrrolo[3,2-d]pyrimidin-6-one | 55.8 | 38.4 | 176.4 |
| 10 | 1-acetyl-4'-amino-5'-(2-aminobenzo[d]oxazol-5-yl)spiro[azetidine-3,7'-cyclopenta[d]pyrimidin]-6' (5'H)-one | 228.9 | >9999 | >9999 |
| 11 | tert-butyl-4'-amino-5'-(2-aminobenzo[d]oxazol-5-yl)-6'-oxo-5',6'-dihydrospiro[azetidine-3,7'-pyrrolo[3,2-d]pyrimidine]-1-carboxylate | 109.7 | | |
| 12 | tert-butyl-4'-amino-5'-(2-aminobenzo[d]oxazol-5-yl)-5',6'-dihydrospiro[azetidine-3,7'-pyrrolo[3,2-d]pyrimidine]-1-carboxylate | 50.5 | 88.1 | 127.9 |
| énantiomère Exemple 4 | 5-(2-amino-benzooxazol-5-yl)-7-methyl-7-methylsulfanylmethyl-6,7-dihydro-5H-pyrrolo[3,2-d]pyrimidin-4-ylamine | 51.6 | 82.1 | 158.6 |
| racémique (Exemples 1 et 2) | 5-(2-amino-benzooxazol-5-yl)-7-isobutyl-7-methyl-6,7-dihydro-5H-pyrrolo[3,2-d]pyrimidin-4-ylamine | 2.4 | 4.2 | 6.3 |
| autre énantiomère Exemple 4 | 5-(2-amino-benzooxazol-5-yl)-7-methyl-7-methylsulfanylmethyl-6,7-dihydro-5H-pyrrolo[3,2-d] pyrimidin-4-ylamine | 8.0 | 4.7 | 8.3 |

| | | | | |
|---|---|---|---|---|
| IC50 : concentration de l'inhibiteur causant 50% d'inhibition. Il s'agit d'un indice pratique d'efficacité. Ki : constante de dissociation du complexe enzyme - inhibiteur. Il indique l'affinité entre l'enzyme et l'inhibiteur (de façon inverse). | | | | |

L'affinité d'un inhibiteur pour une enzyme est donnée par la constante d'inhibition Ki, qui représente la concentration en inhibiteur pour laquelle la moitié des sites enzymatiques sont occupés. Ainsi, l'affinité d'un inhibiteur est d'autant plus grande que le Ki est petit. Cette constante d'inhibition, exprimée en mole par litre correspond aussi à la constante de dissociation du complexe enzyme-inhibiteur.

Considérant les résultats ci-dessus, le composé selon l'exemple 1 apparait présenter la meilleure activité inhibitrice de la kinase mTOR, à la fois sur mTORC1 et mTORC2.

## Revendications

1. Composé inhibiteur de mTOR de formule générale (I) 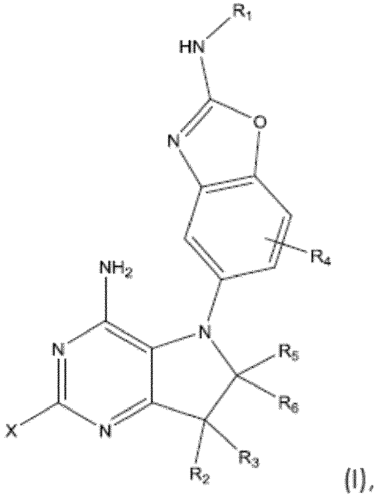 ou un de ses sels pharmaceutiquement acceptables, dans laquelle:
R₁ représente un atome d'hydrogène, un radical alkyle en C₁-C₃, cyclopropyle ou acyle; R₂ et R₃ représentent, indépendamment l'un de l'autre, un atome d'hydrogène, un atome d'halogène choisi parmi Cl ou F, un radical alkyle en C₁-C₆ linéaire ou ramifié, interrompu ou non par un hétéroatome O, S, ou -NR₇, et non substitué ou substitué par un cycloalkyle ou un hétérocycloalkyle en C₃-C₅ ou un cycle aromatique/hétérocycle non substitué ou mono- ou poly- substitué par un atome d'halogène choisi parmi Cl, F ou un radical -OH, méthyle (Me), -OMe, ou forment ensemble un cycle ou un hétérocycloalkyle en C₃-C₆;
étant entendu que R₂ et R₃ ne représentent pas tous deux un atome d'halogène;
avec R₇ représentant un atome d'hydrogène, un radical alkyle en C₁-C₃, acyle, ou carboxyalkyle en C₁-C₄;
R₄ représente un atome d'hydrogène, un atome d'halogène choisi parmi F, Cl, Br, un radical -OR₈, alkyle en C₁-C₃, cyclopropyle,
avec R₈ représentant un atome d'hydrogène, un radical alkyle en C₁-C₃, cyclopropyle ou acyle;
R₅ et R₆ représentent, indépendamment l'un de l'autre, un atome d'hydrogène, un radical alkyle en C₁-C₃, cyclopropyle ou forment ensemble un carbonyle ou un cycle en C₃-C₄; et
X représente un atome d'hydrogène, ou un radical -NH₂, -OH, méthyle.

2. Composé inhibiteur de mTOR de formule (I) selon la revendication 1, ou un de ses sels pharmaceutiquement acceptables, dans laquelle:
R₁ représente un atome d'hydrogène;
R₂ et R₃ représentent, indépendamment l'un de l'autre, un atome d'hydrogène, un radical alkyle en C₁-C₄ linéaire ou ramifié, interrompu ou non par un hétéroatome S ou -NR₇, ou forment ensemble un hétérocycloalkyle en C₄;
avec R₇ représentant un radical acyle, carboxytertiobutyle;
R₄ représente un atome d'hydrogène;
R₅ et R₆ représentent, indépendamment l'un de l'autre, un atome d'hydrogène, un groupe méthyle, ou forment ensemble un carbonyle; et
X représente un atome d'hydrogène.

3. Composé inhibiteur de mTOR de formule (I) selon l'une des revendications 1 ou 2, ou un de ses sels pharmaceutiquement acceptables, dans laquelle:
R₁ représente un atome d'hydrogène;
R₂ représente un groupe méthyle;
R₃ représente un radical alkyle en C₁-C₄ linéaire ou ramifié, interrompu ou non par un hétéroatome S;
R₄ représente un atome d'hydrogène;
R₅ et R₆ représentent un atome d'hydrogène; et
X représente un atome d'hydrogène.

4. Composé inhibiteur de mTOR de formule (I) selon l'une des revendications 1 à 3, choisi parmi les composés suivants:
• 5-(4-Amino-7-isobutyl-7-methyl-6,7-dihydro-5H-pyrrolo[3,2-d]pyrimidin-5-yl)benzo[d]oxazol-2-amine de formule e1 ou formule e2;
• 5-(2-Amino-benzooxazol-5-yl)-7-isobutyl-6,7-dihydro-5H-pyrrolo[3,2-d]pyrimidin-4-ylamine;
• 5-(2-Amino-benzooxazol-5-yl)-7-methyl-7-methylsulfanylmethyl-6,7-dihydro-5H-pyrrolo[3,2-d]pyrimidin-4-ylamine;
• 5-(4-Amino-7,7-diethyl-6,7-dihydro-5H-pyrrolo[3,2-d]pyrimidin-5-yl)benzo[d]oxazol-2-amine;
• 5-(2-Amino-benzooxazol-5-yl)-7,7-dimethyl-6,7-dihydro-5H-pyrrolo[3,2-d]pyrimidin-4-ylamine;
• 5-(2-Amino-benzooxazol-5-yl)-7-methyl-7-propyl-6,7-dihydro-5H-pyrrolo[3,2-d]pyrimidin-4-ylamine;
• 5-(2-Amino-benzooxazol-5-yl)-7,7-diethyl-6-methyl-6,7-dihydro-5H-pyrrolo[3,2-d]pyrimidin-4-ylamine;
• 4-Amino-5-(2-amino-benzooxazol-5-yl)-7,7-dimethyl-5,7-dihydro-pyrrolo[3,2-d]pyrimidin-6-one;
• 1-Acetyl-4'-amino-5'-(2-aminobenzo[d]oxazol-5-yl)spiro[azetidine-3,7'-cyclopenta[d]pyrimidin]-6'(5'H)-one;
• Tert-butyl-4'-amino-5'-(2-aminobenzo[d]oxazol-5-yl)-6'-oxo-5',6'-dihydrospiro[azetidine-3,7'-pyrrolo[3,2-d]pyrimidine]-1-carboxylate;
• Tert-butyl-4'-amino-5'-(2-aminobenzo[d]oxazol-5-yl)-5',6'-dihydrospiro[azetidine-3,7'-pyrrolo[3,2-d]pyrimidine]-1-carboxylate; 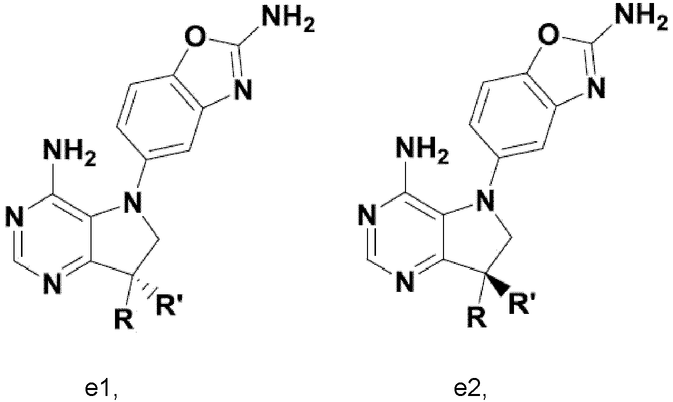
ou un de ses sels pharmaceutiquement acceptables.

5. Composition comprenant, dans un milieu physiologiquement acceptable, un composé inhibiteur de mTOR de formule (I) selon l'une des revendications 1 à 4 ou un de ses sels pharmaceutiquement acceptables.

6. Composition selon la revendication 5, **caractérisée en ce qu'**elle comprend entre 0,001 % et 5% dudit composé en poids du poids total de la composition.

7. Composition selon la revendication 5 ou 6, **caractérisée en ce qu'**elle est sous une forme adaptée pour une administration par voie orale ou topique.

8. Composition selon la revendication 7, **caractérisée en ce qu'**elle est sous une forme adaptée pour une administration par topique.

9. Composition selon l'une des revendications 5 à 8, **caractérisée en ce qu'**elle comprend un autre ingrédient actif, choisi parmi la bétaméthasone dipropionate glycol, clobétasol 17-propionate, halobétasol propionate, amcinonide, desoximétasone, diflucortolone valérate, fluocinonide, halcinonide, mométhasone furoate, triamcinolone acétonide, bétaméthasone valérate, clobétasone 17-butyrate, désonide, hydrocortisone 17-valérate, prédnicarbate, hydrocortisone, hydrocortisone acétate, calcipotriol, calcitriol, adapalène, péroxyde de benzoyle, clindamycine et érythromycine.

10. Composition selon l'une des revendications 5 à 9, pour son utilisation en tant que médicament.

11. Composition selon la revendication 10, pour son utilisation dans le traitement des affections dermatologiques liées à un trouble de la kératinisation avec une composante proliférative, inflammatoire et/ou immuno-allergique telles que le psoriasis, la dermatite atopique, la kératose actinique, ou l'acné.

12. Composition selon la revendication 11, pour son utilisation dans le traitement de la dermatite atopique.

13. Composition selon la revendication 12, pour son utilisation dans le traitement de la composante inflammatoire de la dermatite atopique.

## Patentansprüche

1. mTOR-Inhibitor-Verbindung der allgemeinen Formel (I) 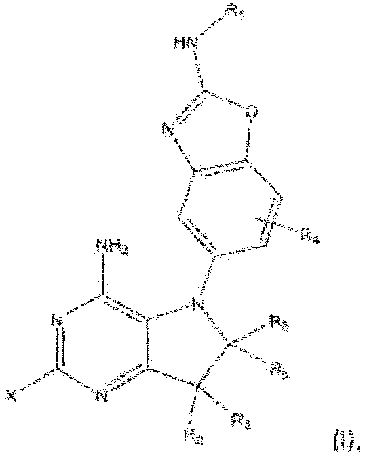 oder ein pharmazeutisch verträgliches Salz davon, wobei:
R₁ ein Wasserstoffatom, einen C₁-C₃-Alkyl-, Cyclopropyl- oder Acylrest darstellt;
R₂ und Rs unabhängig voneinander ein Wasserstoffatom, ein Halogenatom, das aus Cl oder F ausgewählt ist, einen linearen oder verzweigten C₁-C₆-Alkylrest darstellen, der durch ein O-, S- oder -NR₇-Heteroatom unterbrochen ist oder nicht und substituiert ist oder nicht durch ein C₃-C₅-Cycloalkyl oder -Heterocycloalkyl oder einen aromatischen Zyklus/Heterozyklus, der nicht substituiert ist oder durch ein Halogenatom mono- oder polysubstituiert ist, das aus Cl, F oder einem -OH-, Methyl(Me)-, -OMe-Rest ausgewählt ist, oder zusammen einen C₃-C₆-Zyklus oder ein -Heterocycloalkyl bilden;
wobei vorausgesetzt wird, dass R₂ und Rs nicht beide ein Halogenatom darstellen;
wobei R₇ ein Wasserstoffatom, einen C₁-C₃-Alkyl-, Acyl- oder C₁-C₄-Carboxyalkylrest darstellt;
R₄ ein Wasserstoffatom, ein Halogenatom, das aus F, Cl, Br ausgewählt ist, einen - ORs-, C₁-C₃-Alkyl-, Cyclopropylrest darstellt;
wobei R₈ ein Wasserstoffatom, einen C₁-C₃-Alkyl-, Cyclopropyl- oder Acylrest darstellt;
R₅ und R₆ unabhängig voneinander ein Wasserstoffatom, einen C₁-C₃-Alkyl-, Cyclopropylrest darstellen oder zusammen ein Carbonyl oder einen C₃-C₄-Zyklus bilden; und
X ein Wasserstoffatom oder einen -NH₂-, -OH-, Methylrest darstellt.

2. mTOR-Inhibitor-Verbindung der Formel (I) nach Anspruch 1 oder ein pharmazeutisch verträgliches Salz davon, wobei:
R₁ ein Wasserstoffatom darstellt;
R₂ und Rs unabhängig voneinander ein Wasserstoffatom, einen linearen oder verzweigten C₁-C₄-Alkylrest darstellen, der durch ein S- oder -NR₇-Heteroatom unterbrochen ist oder nicht, oder zusammen ein C₄-Heterocycloalkyl bilden;
wobei R₇ einen Acyl-, Carboxytertiobutylrest darstellt;
R₄ ein Wasserstoffatom darstellt;
R₅ und R₆ unabhängig voneinander ein Wasserstoffatom, eine Methylgruppe darstellen oder zusammen ein Carbonyl bilden; und
X ein Wasserstoffatom darstellt.

3. mTOR-Inhibitor-Verbindung der Formel (I) nach einem der Ansprüche 1 oder 2 oder ein pharmazeutisch verträgliches Salz davon, wobei:
R₁ ein Wasserstoffatom darstellt;
R₂ eine Methylgruppe darstellt;
R₃ einen linearen oder verzweigten C₁-C₄-Alkylrest darstellt, der durch ein S-Heteroatom unterbrochen ist oder nicht;
R₄ ein Wasserstoffatom darstellt;
R₅ und R₆ ein Wasserstoffatom darstellen; und
X ein Wasserstoffatom darstellt.

4. mTOR-Inhibitor-Verbindung der Formel (I) nach einem der Ansprüche 1 bis 3, die aus den folgenden Verbindungen ausgewählt ist:
• 5-(4-Amino-7-isobutyl-7-methyl-6,7-dihydro-5H-pyrrolo[3,2-d]pyrimidin-5-yl)benzo[d]oxazol-2-amin der Formel e1 oder Formel e2;
• 5-(2-Amino-benzooxazol-5-yl)-7-isobutyl-6,7-dihydro-5H-pyrrolo[3,2-d]pyrimidin-4-ylamin;
• 5-(2-Amino-benzooxazol-5-yl)-7-methyl-7-methylsulfanyimethyl-6,7-dihydro-5H-pyrrolo[3,2-d]pyrimidin-4-ylamin;
• 5-(4-Amino-7,7-diethyl-6,7-dihydro-5H-pyrrolo[3,2-d]pyrimidin-5-yl)benzo[d]oxazol-2-amin;
• 5-(2-Amino-benzooxazol-5-yl)-7,7-dimethyl-6,7-dihydro-5H-pyrrolo[3,2-d]pyrimidin-4-ylamin;
• 5-(2-Amino-benzooxazol-5-yl)-7-methyl-7-propyl-6,7-dihydro-5H-pyrrolo[3,2-d]pyrimidin-4-ylamin;
• 5-(2-Amino-benzooxazol-5-yl)-7,7-diethyl-6-methyl-6,7-dihydro-5H-pyrrolo[3,2-d]pyrimidin-4-ylamin;
• 4-Amino-5-(2-amino-benzooxazol-5-yl)-7,7-dimethyl-5,7-dihydro-pyrrolo[3,2-d]pyrimidin-6-on;
• 1-Acetyl-4'-amino-5'-(2-aminobenzo[d]oxazol-5-yl)spiro[azetidin-3,7'-cyclopenta[d]pyrimidin]-6'(5'H)-on;
• Tert-butyl-4'-amino-5'-(2-aminobenzo[d]oxazol-5-yl)-6'-oxo-5',6'-dihydrospiro[azetidin-3,7'-pyrrolo[3,2-d]pyrimidin]-1-carboxylat;
• Tert-butyl-4'-amino-5'-(2-aminobenzo[d]oxazol-5-yl)-5',6'-dihydrospiro[azetidin-3,7'-pyrrolo[3,2-d]pyrimidin]-1-carboxylat; 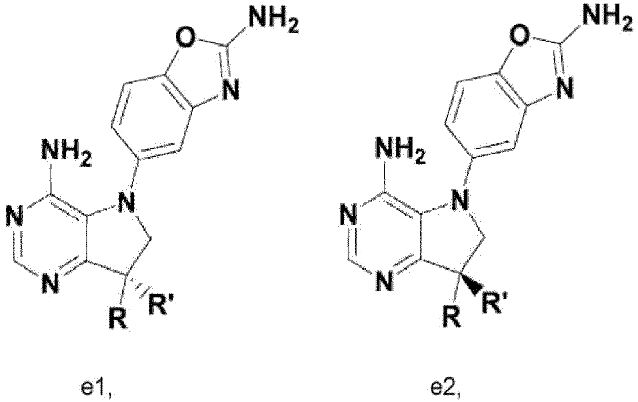
oder ein pharmazeutisch verträgliches Salz davon.

5. Zusammensetzung, die in einem physiologisch verträglichen Medium eine mTOR-Inhibitor-Verbindung der Formel (I) nach einem der Ansprüche 1 bis 4 oder ein pharmazeutisch verträgliches Salz davon umfasst.

6. Zusammensetzung nach Anspruch 5, **dadurch gekennzeichnet, dass** sie zwischen 0,001 Gew.-% und 5 Gew.-% der besagten Verbindung des Gesamtgewichts der Zusammensetzung umfasst.

7. Zusammensetzung nach Anspruch 5 oder 6, **dadurch gekennzeichnet, dass** sie in einer Form vorliegt, die für eine orale oder topische Verabreichung geeignet ist.

8. Zusammensetzung nach Anspruch 7, **dadurch gekennzeichnet, dass** sie in einer Form vorliegt, die für eine topische Verabreichung geeignet ist.

9. Zusammensetzung nach einem der Ansprüche 5 bis 8, **dadurch gekennzeichnet, dass** sie einen anderen Wirkstoff umfasst, der aus Betamethasondipropionatglykol, Clobetasol-17-Propionat, Halobetasolpropionat, Amcinonid, Desoximetason, Diflucortolonvalerat, Fluocinonid, Halcinonid, Momethasonfuroat, Triamcinolonacetonid, Betamethasonvalerat, Clobetason-17-Butyrat, Desonid, Hydrocortison-17-Valerat, Prednicarbat, Hydrocortison, Hydrocortisonacetat, Calcipotriol, Calcitriol, Adapalen, Benzoylperoxid, Clindamycin und Erythromycin ausgewählt ist.

10. Zusammensetzung nach einem der Ansprüche 5 bis 9 zu ihrer Verwendung als Medikament.

11. Zusammensetzung nach Anspruch 10 zu ihrer Verwendung bei der Behandlung von dermatologischen Erkrankungen, die mit einer Keratinisierungsstörung mit einer proliferativen, entzündlichen und/oder immunallergischen Komponente wie die Psoriasis, atopische Dermatitis, aktinische Keratose oder Akne verbunden sind.

12. Zusammensetzung nach Anspruch 11 zu ihrer Verwendung bei der Behandlung der atopischen Dermatitis.

13. Zusammensetzung nach Anspruch 12 zu ihrer Verwendung bei der Behandlung der entzündlichen Komponente der atopischen Dermatitis.

## Claims

1. mTOR-inhibiting compound of general formula (I) 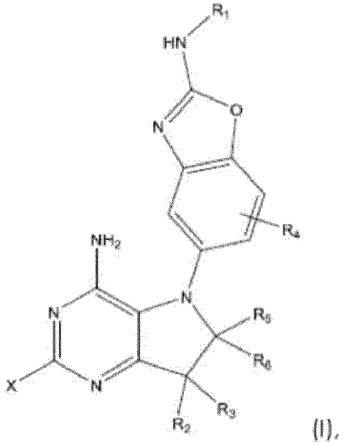 or a pharmaceutically acceptable salt thereof, wherein:
R₁ represents a hydrogen atom, a C₁-C₃ alkyl, cyclopropyl or acyl radical;
R₂ and R₃ represent, independently of each other, a hydrogen atom, a halogen atom selected from Cl or F, a linear or branched C₁-C₆ alkyl radical, interrupted or not by an O, S, or -NR₇ heteroatom, and unsubstituted or substituted with a C₃-C₅ cycloalkyl or heterocycloalkyl or an aromatic ring/heterocycle unsubstituted or mono- or polysubstituted with a halogen atom selected from Cl, F or a -OH, methyl (Me), -OMe radical or together form a C₃-C₆ ring or heterocycloalkyl;
it being understood that R₂ and R₃ do not both represent a halogen atom;
with R₇ representing a hydrogen atom, a C₁-C₃ alkyl, acyl, or C₁-C₄ carboxyalkyl radical;
R₄ represents a hydrogen atom, a halogen atom selected from F, Cl, Br, an -OR₈, C₁-C₃ alkyl, cyclopropyl radical;
with R₈ representing a hydrogen atom, a C₁-C₃ alkyl, cyclopropyl or acyl radical;
R₅ and R₆ represent, independently of each other, a hydrogen atom, a C₁-C₃ alkyl, cyclopropyl radical or together form a carbonyl or C₃-C₄ ring; and
X represents a hydrogen atom, or an -NH₂, -OH, methyl radical.

2. mTOR-inhibiting compound of formula (I) according to claim 1, or a pharmaceutically acceptable salt thereof, wherein:
R₁ represents a hydrogen atom;
R₂ and R₃ represent, independently of each other, a hydrogen atom, a linear or branched C₁-C₄ alkyl radical, interrupted or not by an S or -NR₇ heteroatom, or together form a C₄ heterocycloalkyl;
with R₇ representing an acyl or carboxy-tert-butyl radical;
R₄ represents a hydrogen atom;
R₅ and R₆ represent, independently of each other, a hydrogen atom, a methyl group, or together form a carbonyl; and
X represents a hydrogen atom.

3. mTOR-inhibiting compound of formula (I) according to one of claims 1 or 2, or a pharmaceutically acceptable salt thereof, wherein:
R₁ represents a hydrogen atom;
R₂ represents a methyl group;
R₃ represents a linear or branched C₁-C₄ alkyl radical, interrupted or not by an S heteroatom;
R₄ represents a hydrogen atom;
R₅ and R₆ represent a hydrogen atom; and
X represents a hydrogen atom.

4. mTOR-inhibiting compound of formula (I) according to one of claims 1 to 3, selected from the following compounds:
• 5-(4-Amino-7-isobutyl-7-methyl-6,7-dihydro-5H-pyrrolo[3,2-d]pyrimidin-5-yl)benzo[d]oxazol-2-amine of formula e1 or formula e2;
• 5-(2-Amino-benzooxazol-5-yl)-7-isobutyl-6,7-dihydro-5H-pyrrolo[3,2-d]pyrimidin-4-ylamine;
• 5-(2-Amino-benzooxazol-5-yl)-7-methyl-7-methylsulfanyl methyl-6,7-dihydro-5H-pyrrolo[3,2-d]pyrimidin-4-ylamine;
• 5-(4-Amino-7,7-diethyl-6,7-dihydro-5H-pyrrolo[32-d]pyrimidin-5-yl)benzo[d]oxazol-2-amine;
• 5-(2-Amino-benzooxazol-5-yl)-7,7-dimethyl-6,7-dihydro-5H-pyrrolo[3,2-d]pyrimidin-4-ylamine;
• 5-(2-Amino-benzooxazol-5-yl)-7-methyl-7-propyl-6,7-dihydro-5H-pyrrolo[3,2-d]pyrimidin-4-ylamine;
• 5-(2-Amino-benzooxazol-5-yl)-7,7-diethyl-6-methyl-6,7-dihydro-5H-pyrrolo[3,2-d]pyrimidin-4-ylamine;
• 4-Amino-5-(2-amino-benzooxazol-5-yl)-7,7-dimethyl-5,7-dihydro-pyrrolo[3,2-d]pyrimidin-6-one;
• 1-Acetyl-4'-amino-5'-(2-aminobenzo[d]oxazol-5-yl)spiro[azetidine-3,7'-cyclopenta[d]pyrimidin]-6'(5'H)- one;
• Tert-butyl-4'-amino-5'-(2-aminobenzo[d]oxazol-5-yl)-6'-oxo-5',6'-dihydrospiro[azetidine-3,7'-pyrrolo[3 ,2-d]pyrimidine]-1-carboxylate;
• Tert-butyl-4'-amino-5'-(2-aminobenzo[d]oxazol-5-yl)-5',6'-dihydrospiro[azetidine-3,7'-pyrrolo[3,2-d]pyrimidine]-1-carboxylate; 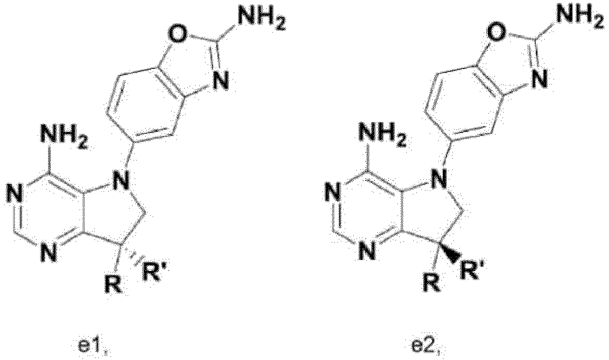
or a pharmaceutically acceptable salt thereof.

5. Composition comprising, in a physiologically acceptable medium, an mTOR-inhibiting compound of formula (I) according to one of claims 1 to 4 or a pharmaceutically acceptable salt thereof.

6. Composition according to claim 5, **characterized in that** it comprises between 0.001% and 5% of said compound by weight relative to the total weight of the composition.

7. Composition according to claim 5 or 6, **characterized in that** it is in a form suitable for oral or topical administration.

8. Composition according to claim 7, **characterized in that** it is in a form suitable for topical administration.

9. Composition according to one of claims 5 to 8, **characterized in that** it comprises another active ingredient, selected from betamethasone dipropionate glycol, clobetasol 17-propionate, halobetasol propionate, amcinonide, desoximetasone, diflucortolone valerate, fluocinonide, halcinonide, momethasone furoate, triamcinolone acetonide, betamethasone valerate, clobetasone 17-butyrate, desonide, hydrocortisone 17-valerate, prednicarbate, hydrocortisone, hydrocortisone acetate, calcipotriol, calcitriol, adapalene, benzoyl peroxide, clindamycin and erythromycin.

10. Composition according to one of claims 5 to 9, for use as a medicament.

11. Composition according to claim 10, for use in the treatment of dermatological conditions linked to a keratinization disorder with a proliferative, inflammatory and/or immuno-allergic component such as psoriasis, atopic dermatitis, actinic keratosis, or acne.

12. Composition according to claim 11, for use in the treatment of atopic dermatitis.

13. Composition according to claim 12, for use in the treatment of the inflammatory component in atopic dermatitis.
